# EUROPEAN PATENT SPECIFICATION

(11) **EP 0 620 279 B1**
(45) Date of publication and mention of the grant of the patent: **05.02.2003**
(21) Application number: 94104777.1
(22) Date of filing: 14.04.1983
(51) Int. Cl.: C12N 15/58, C12N 9/72, C12N 15/79, C12N 5/10, C12N 1/19, A61K 38/46

(54) **Preparation of functional human urokinase proteins**
Herstellung von funktionellen, menschlichen Urokinaseproteinen
Préparation de protéines fonctionnelles d'urokinase humaine

(30) Priority: 15.04.1982 US 368773; 14.03.1983 US 474930
(43) Date of publication of application: 19.10.1994
(62) Divisional of application: 83103629.8
(73) Proprietor: GENENTECH, INC., South San Francisco, CA 94080-4990 (US)
(72) Inventor: Heyneker, Herbert Louis, Hillsborough, CA 94010 (US); Holmes, William Evans, Pacifica, CA 94044 (US); Vehar, Gordon Alan, San Carlos, CA 94070 (US)
(74) Representative: Armitage, Ian Michael

(56) References cited:
- EP-A- 0 037 687
- EP-A- 0 049 619
- CHEMICAL ABSTRACTS, vol. 97, no. 13, 27 September 1982, Columbus, Ohio, US; abstract no. 104934b, P.P.HUNG 'Expression in Escherichia coli of a biologically active human enzyme,urokinase.' page 143 ; & PROC. BATTELLE CONF. GENET. ENG. vol. 5 , 1981 pages 68 - 90
- CHEMICAL ABSTRACTS, vol. 96, no. 5, 1 February 1982, Columbus, Ohio, US; abstract no. 29397c, A. BOLLEN ET AL. 'Expression in Escherichia coli of urokinase antigenic determinants' page 145 ; & BIOCHEM. BIOPHYS. RES. COMMUN. vol. 103, no. 2 , 1981 pages 391 - 401

## Description

### Reference to Related Application

This is a continuation-in-part of application USSN 06/368773, filed April 15, 1982.

### Field of the Invention

The present invention relates to human urokinase, to novel forms and compositions thereof and particularly to means and methods for the preparation in vitro of functional protein species of human urokinase.

The present invention is based in part on the discovery of the DNA sequence and deduced amino acid sequence of native urokinase as well as associated portions of the urokinase molecule found to be the functional bioactive moieties. This discovery enabled the production of urokinase in various forms via the application of recombinant DNA technology, in turn enabling the production of sufficient quality and quantity of materials with which to conduct requisite biological testing identifying the biologically functional, hence useful moieties of the molecule. Having determined such, it was possible to tailor-make functional species of urokinase via genetic manipulation and in vitro processing, arriving efficiently at hitherto unobtainable commercially efficacious amounts of active products. This invention is directed to these associated embodiments in all respects.

The publications and other materials hereof used to illuminate the background of the invention, and in particular cases, to provide additional details concerning its practice are incorporated herein by reference, and for convenience, are numerically referenced in the following text and respectively grouped in the appended bibliography.

### Background of the Invention

### A. Human Urokinase

The fibrinolytic system is in a dynamic equilibrium with the coagulation system, maintaining an intact, patent vascular bed. The coagulation system deposits fibrin as a matrix serving to restore a hemostatic condition. The fibrinolytic system removes the fibrin network after the hemostatic condition is achieved. The fibrinolytic process is brought about by the proteolytic enzyme plasmin that is generated from a plasma protein precursor plasminogen. Plasminogen is converted to plasmin through activation by an activator.

Urokinase is one such activator. It and another activator, streptokinase, are currently commercially available. Both are indicated for the treatment of acute vascular diseases such as myocardial infarct, stroke, pulmonary embolism, deep vein thrombosis, peripheral arterial occlusion and other venous thromboses. Collectively, these diseases account for major health hazards and risks.

The underlying etiological basis for these diseases points to either a partial, or in severe cases, total occlusion of a blood vessel by a blood clot -- thrombus or thromboembolus. Traditional anticoagulant therapy, as with heparin and coumarin, does nothing to directly enhance dissolution of thrombi or thromboemboli. Streptokinase and urokinase have enjoyed practical and effective use as thrombolytic agents. Until now, however, each has suffered from severe limitations. Neither has demonstrated a high affinity for fibrin; consequently, both activate circulating and fibrin-bound plasminogen relatively indiscriminately. The plasmin formed in circulating blood is neutralized rather quickly and lost for useful thrombolysis. Residual plasmin will degrade several clotting factor proteins, for example, fibrinogen, Factor V and Factor VIII, causing a hemorrhagic potential. In addition, streptokinase is strongly antigenic and patients with high antibody titers respond inefficiently to treatment and cannot remain on continuous treatment. Urokinase therapy is expensive, owing to its involved isolation from human urine or tissue culture, and it therefore is not generally accepted in clinical practice. Urokinase has been the subject of numerous investigations -- See, for example, references 1-9. Presently available urokinase, as defined, is isolated from human urine or tissue culture, e.g. kidney cells (9A,9B).

The urokinase molecule exists in several biologically active forms - high molecular weight (ca. 54000 daltons) and low molecular weight (ca. 33000 daltons), each composed of single chain or two chain material. The low molecular weight form is derived from the high molecular weight form by enzymatic cleavage. Biologically active material contains the so-called serine protease portion linked, in active form, to a second chain via a disulfide bond. Any activity ascribed to the high molecular weight material is believed to be due to the similar presence of these two connected chains, the strategic disulfide bond and interruption in the sequence doubtless being located in the serine protease portion of the overall molecules (See Figure A). In any event, until the present invention, the identity, and hence function, of the ca. 21000 dalton residue was unknown and the assignment of activity to one or another of the known moieties of urokinase was not uncontrovertedly possible.

Recently, there was a report of another form of urokinase peptide having low, but specific activity (10, 10A). It was speculated that this material corresponds to native urokinase, a preform of the previously isolated active species described above, most probably consisting of a single chain.

Previous attempts to clone the requisite gene for urokinase with attendant hopes of attaining expression in a microbial host were not believed successful (11, 11A). See also (6).

It was perceived that the application of recombinant DNA and associated technologies, after all, would be a most effective way of providing the requisite large quantities of high quality, bioactive human urokinase, essentially free of other human protein, and derivatives thereof that retain functional bioactivity, thus admitting of the use of such materials clinically in the treatment of various vascular conditions or diseases.

### B. Recombinant DNA/Protein Biochemistry Technology

Recombinant DNA technology has reached the age of some sophistication. Molecular biologists are able to recombine various DNA sequences with some facility, creating new DNA entities capable of producing copious amounts of exogenous protein product in transformed microbes and cell cultures. The general means and methods are in hand for the in vitro ligation of various blunt ended or "sticky" ended fragments of DNA, producing potent expression vehicles useful in transforming particular organisms, thus directing their efficient synthesis of desired exogenous product. However, on an individual product basis, the pathway remains somewhat tortuous and the science has not advanced to a stage where regular predictions of success can be made. Indeed, those who portend successful results without the underlying experimental basis, do so with considerable risk of inoperability.

DNA recombination of the essential elements, i.e., an origin of replication, one or more phenotypic selection characteristics, an expression promoter, heterologous gene insert and remainder vector, generally is performed outside the host cell. The resulting recombinant replicable expression vehicle, or plasmid, is introduced into cells by transformation and large quantities of the recombinant vehicle obtained by growing the transformant. Where the gene is properly inserted with reference to portions which govern the transcription and translation of the encoded DNA message, the resulting expression vehicle is useful to actually produce the polypeptide sequence for which the inserted gene codes, a process referred to as expression. The resulting product may be obtained by lysing, if necessary, the host cell, in microbial systems, and recovering the product by appropriate purification from other proteins.

In practice, the use of recombinant DNA technology can express entirely heterologous polypeptides--so-called direct expression--or alternatively may express a heterologous polypeptide fused to a portion of the amino acid sequence of a homologous polypeptide. In the latter cases, the intended bioactive product is sometimes rendered bioinactive within the fused, homologous/heterologous polypeptide until it is cleaved in an extracellular environment. See references (12) and (13).

Similarly, the art of cell or tissue cultures for studying genetics and cell physiology is well established. Means and methods are in hand for maintaining permanent cell lines, prepared by successive serial transfers from isolate normal cells. For use in research, such cell lines are maintained on a solid support in liquid medium, or by growth in suspension containing support nutriments. Scale-up for large preparations seems to pose only mechanical problems. For further background, attention is directed to references (14) and (15).

Likewise, protein biochemistry is a useful, indeed necessary, adjunct in biotechnology. Cells producing the desired protein also produce hundreds of other proteins, endogenous products of the cell's metabolism. These contaminating proteins, as well as other compounds, if not removed from the desired protein, would prove toxic if administered to an animal or human in the course of therapeutic treatment with desired protein. Hence, the techniques of protein biochemistry come to bear, allowing the design of separation procedures suitable for the particular system under consideration and providing a homogeneous product safe for intended use. Protein biochemistry also proves the identity of the desired product characterizing it and ensuring that the cells have produced it faithfully with no alterations or mutations. This branch of science is also involved in the design of bioassays, stability studies and other procedures necessary to apply before successful clinical studies and marketing can take place.

### Summary of the Invention

The present invention is based upon the discovery that recombinant DNA/protein biochemistry technology can be used to successfully produce human urokinase in the form of biologically functional, tailored species. This invention relates to active urokinase protein suitable for use, in all of its forms, in the prophylactic or therapeutic treatment of human beings for various vascular conditions or diseases. Each of its forms includes the bioactive moiety, to wit, the enzymatic portion of native material believed to reside in a 2-chain region comprising the serine protease portion. A series of urokinase active products can be prepared, either directly in bioactive form or notably in a form available for in vitro processing to result in bioactive product. This invention also relates to the means and methods for producing full length native urokinase molecules particularly in bioactive or bioactivatable form, having the potential added advantage of specific affinity for fibrin not demonstrated until now with any urokinase product isolated from natural sources. Thus provided is human urokinase product having the potential new property of specific activity toward tangible, extant thrombi. The products being produced by cell culture harboring recombinant DNA encoding respective product entity, the facilities are now at hand to produce human urokinase in a much more efficient manner than has been possible and in forms exhibiting enhanced biologically significant properties. In addition, depending upon the host cell, the urokinase activator hereof may contain associated glycosylation to a greater or lesser extent compared with native material.

The present invention relates to the human urokinase products thus produced and provides the means and methods of production. In one aspect, the present invention provides replicable DNA expression vehicles harboring gene sequences encoding the enzymatic portion of human urokinase in expressible form, as defined in claim 10. In another aspect, the present invention provides cells as defined in claim 11, transformed with the expression vehicles described above.

The present invention also provides various processes useful for preparing DNA expression vehicles comprising said urokinase gene sequences, microorganism strains and cell cultures and specific embodiments thereof. Still further, this invention is directed to the preparation of fermentation cultures of said microorganisms and cell cultures, and the production of glycosylated polypeptide therefrom.

Reference herein to the expression "human urokinase" connotes polypeptide in bioactive form, produced by microbial or cell culture or optional in vitro processing and comprising the enzymatic portion corresponding to native material. Human urokinase, as referred to herein, is thus 1) in full length, in contradistinction to material hitherto isolated from natural sources or 2) in other, bioactive forms bearing the sites of the enzymatic portion found essential for plasminogen activation or 3) having a methionine first amino acid or a signal polypeptide or conjugated polypeptide other than the signal polypeptide fused at the N-terminus of the enzymatic portion, the methionine, signal or conjugate polypeptide being specifically cleavable in an intra- or extracellular environment (See reference 12). In any event, the thus produced human urokinase polypeptides are recovered and purified to levels fitting them for use in the treatment of various cardiovascular conditions or diseases.

### Description of Preferred Embodiments

### A. Microorganisms/Cell Cultures

### 1. Bacterial Strains/Promoters

The work described herein was performed employing, inter alia, the microorganism E. coli K-12 strain GM 48 (thr⁻, leu⁻, B₁⁻, lacY1, gal K12, gal T22, ara 14, ton A31, tsx 78, Sup E44, dam 3, dcm 6) deposited with the American Type Culture Collection on April 9, 1982 (ATCC No. 39099) and E. coli K-12 strain 294 (end A, thi⁻, hsr⁻, ₖhsm⁺), described in reference (16), deposited with the American Type Culture Collection, ATCC Accession No. 31446, on October 28, 1978. However, various other microbial strains are useful, including known E. coli strains such as E. coli B, E. coli X 1776 (ATCC No. 31537, deposited July 3, 1979) and E. coli W 3110 (F⁻, λ⁻, protrophic) (ATCC No. 27325 deposited February 10, 1972), or other microbial strains many of which are deposited and (potentially) available from recognized microorganism depository institutions, such as the American Type Culture Collection (ATCC)--cf. the ATCC catalogue listing. See (17). These other microorganisms include, for example, Bacilli such as Bacillus subtilis and other enterobacteriaceae among which can be mentioned as examples Salmonella typhimurium, Serratia marcesans, and Pseudomonas. utilizing plasmids that can replicate and express heterologous gene sequences therein.

As examples, the beta lactamase and lactose promoter systems have been advantageously used to initiate and sustain microbial production of heterologous polypeptides. Details relating to the make-up and construction of these promoter systems can be had by reference to (18) and (19). More recently, a system based upon the tryptophan pathway, the so-called trp promoter system, has been developed. Details relating to the make-up and construction of this system can be had by reference to (20) and (21). Numerous other microbial promoters have been discovered and utilized and details concerning their nucleotide sequences, enabling a skilled worker to ligate them functionally within plasmid vectors, have been published. -- See (22).

### 2. Yeast Strains/Yeast Promoters

The expression system hereof may also employ a plasmid which is capable of selection and replication in either or both E. coli and/or yeast, Saccharomyces cerevisiae. For selection in yeast the plasmid may contain the TRP1 gene (23, 24, 25) which complements (allows for growth in the absence of tryptophan) yeast containing mutations in this gene found on chromosome IV of yeast (26). A useful strain is strain RH218 (27) deposited at the American Type Culture Collection without restriction on December 8, 1980. (ATCC No. 44076). However, it will be understood that any Saccharomyces cerevisiae strain containing a mutation which makes the cell trp1 should be an effective environment for expression of the plasmid containing the expression system, e.g., strain pep4-1 (28). This tryptophan auxotroph strain also has a point mutation in TRP1 gene.

When placed on the 5' side of a non-yeast gene the 5'-flanking DNA sequence (promoter) from a yeast gene (for alcohol dehydrogenase 1) can promote the expression of a foreign gene in yeast when placed in a plasmid used to transform yeast. Besides a promoter, proper expression of a non-yeast gene in yeast requires a second yeast sequence placed at the 3'-end of the non-yeast gene on the plasmid so as to allow for proper transcription termination and polyadenylation in yeast. In the preferred embodiments, the 5'-flanking sequence of the yeast 3-phosphoglycerate kinase gene (29) is placed upstream from the structural gene followed again by DNA containing termination - polyadenylation signals, for example, the TRP1 (23-25) gene or the PGK (29) gene.

Because yeast 5'-flanking sequence (in conjunction with 3' yeast termination DNA) (infra) can function to promote expression of foreign genes in yeast, it seems likely that the 5'-flanking sequences of any yeast gene could be used for the expression of important gene products, e.g., glycolytic genes such as e.g., enolase, glyceraldehyde - 3-phosphate dehydrogenase, hexokinase, pyruvate decarboxylase, phosphofructokinase, glucose - 6-phosphate isomerase, 3-phosphoglycerate mutase, pyruvate kinase, triosephosphate isomerase, phosphoglucose isomerase, and glucokinase. Any of the 3'-flanking sequences of these genes could also be used for proper termination and mRNA polyadenylation in such an expression system.

Finally, many yeast promoters also contain transcriptional control so they may be turned off or on by variation in growth conditions. Some examples of such yeast promoters are the genes that produce the following proteins: Alcohol dehydrogenase II, acid phosphatase, degradative enzymes associated with nitrogen metabolism, glyceraldehyde -3-phosphate dehydrogenase, and enzymes responsible for maltose and galactose utilization (30). Such a control region would be very useful in controlling expression of protein product - especially when their production is toxic to yeast. It should also be possible to combine the control region of one 5'-flanking sequence with a 5'-flanking sequence containing a promoter from a highly expressed gene. This would result in a hybrid promoter and should be possible since the control region and the promoter appear to be physically distinct DNA sequences.

### 3. Cell Culture Systems/Cell Culture Vectors

Propogation of vertebrate cells in culture (tissue culture) has become a routine procedure in recent years. (See 31). A useful host for the production of heterologous protein is the COS-7 line of monkey kidney fibroblasts (32). However, the present invention could be practiced in any cell line that is capable of the replication and expression of a compatible vector, e.g., WI38, BHK, 3T3, CHO, VERO, and HeLa cell lines. Additionally, what is required of the expression vector is an origin of replication and a promoter located in front of the gene to be expressed, along with any necessary ribosome binding sites, RNA splice sites, polyadenylation site, and transcriptional terminator sequences. It will be understood that this invention, although described herein in terms of a preferred embodiment, should not be construed as limited to these sequences. For example, the origin of replication of other viral (e.g., Polyoma, Adeno, VSV, BPV, and so forth) vectors could be used, as well as cellular origins of DNA replication which could function in a nonintegrated state.

In such vertebrate cell hosts, the genetic expression vector for a urokinase product polypeptide hereof may also contain a secondary genetic coding sequence under the control of the same promoter. The secondary sequence provides for a convenient screening marker, both for transformants in general, and for transformants showing high expression levels for the primary sequence, as well as serving as a control device whereby the expression of the desired urokinase polypeptide can be regulated, most frequently enhanced.

This is particularly significant as the two proteins are produced separately in mature form. While both DNA coding sequences are controlled by the same transcriptional promoter, so that a fused message (mRNA) is formed, they are separated by a translational stop signal for the first and start signal for the second, so that two independent proteins result.

It has been recognized that environmental conditions are often effective in controlling the quantity of particular enzymes that are produced by cells under certain growth conditions. In a preferred embodiment, advantage is taken of the sensitivity of certain cells to methotrexate (MTX) which is an inhibitor of dihydrofolate reductase (DHFR). DHFR is an enzyme which is required, indirectly, in synthesis reactions involving the transfer of one carbon units. Lack of DHFR activity results in inability of cells to grow except in the presence of those compounds which otherwise require transfer of one carbon units for their synthesis. Cells lacking DHFR, however, will grow in the presence of a combination of glycine, thymidine and hypoxanthine.

Cells which normally produce DHFR are known to be inhibited by methotrexate. Most of the time, addition of appropriate amounts of methotrexate to normal cells will result in the death of the cells. However, certain cells appear to survive the methotrexate treatment by making increased amounts of DHFR, thus exceeding the capacity of the methotrexate to inhibit this enzyme. It has been shown previously that in such cells, there is an increased amount of messenger RNA coding for the DHFR sequence. This is explained by assuming an increase in the amount of DNA in the genetic material coding for this messenger RNA. In effect, apparently the addition of methotrexate causes gene amplification of the DHFR gene. Genetic sequences which are physically connected with the DHFR sequence although not regulated by the same promoter are also amplified. Consequently, it is possible to use the amplification of the DHFR gene resulting from methotrexate treatment to amplify concomitantly the gene for another protein, in this case, the desired urokinase polypeptide.

Moreover, if the host cells into which the secondary sequence for DHFR is introduced are themselves DHFR deficient, DHFR also serves as a convenient marker for selection of cells successfully transfected. If the DHFR sequence is effectively connected to the sequence for the desired peptide, this ability serves as a marker for successful transfection with the desired sequence as well.

### B. Vector Systems

### 1. Expression in Bacterial Systems

Expression plasmids for bacterial use, e.g., E. coli are commonly derived using BR322 (37) as vector and appropriately inserting the heterologous gene sequence together with translational start and stop signals in operable reading phase with a functional promoter, taking advantage of common or synthetically created restriction sites. The vector will carry one or more phenotypic selection characteristic genes and an origin of replication to insure amplification within the host. Again, the heterologous insert can be aligned so as to be expressed together with a fused presequence, derivable for example from the trp system genes.

### 2. Expression in Yeast

To express a heterologous gene such as the cDNA for human urokinase in yeast, it is necessary to construct a plasmid vector containing four components. One component is the part which allows for transformation of both E. coli and yeast and thus must contain a selectable gene from each organism. This can be the gene for ampicillin resistance from E. coli and the gene TRP1 from yeast.) This component also requires an origin of replication from both organisms to be maintained as a plasmid DNA in both organisms. This can be the E. coli origin from pBR322 and the ars1 origin from chromosome III of yeast.)

A second component of the plasmid is a 5'-flanking sequence from a yeast gene to promote transcription of a downstream-placed structural gene. The 5'-flanking sequence can be that from the yeast 3-phospho- glycerate kinase (PGK) gene. The fragment is constructed in such a way so as to remove the ATG of the PGK structural sequence, replaced with a sequence containing alternative restriction sites, such as XbaI and EcoRI restriction sites, for convenient attachment of this 5'-flanking sequence to the structural gene.

A third component of the system is a structural gene constructed in such a manner that it contains both an ATG translational start and translational stop signals.

A fourth component is a yeast DNA sequence containing the 3'-flanking sequence of a yeast gene, which contains the proper signals for transcription termination and polyadenylation.

### 3. Expression in Mammalian Cell Culture

The strategy for the synthesis of heterologous peptide in mammalian cell culture relies on the development of a vector capable of both autonomous replication and expression of a foreign gene under the control of a heterologous transcriptional unit. The replication of this vector in tissue culture is accomplished by providing a DNA replication origin (such as from SV40 virus), and providing helper function (T antigen) by the introduction of the vector into a cell line endogenously expressing this antigen (33, 34). The late promoter of SV40 virus precedes the structural gene and ensures the transcription of the gene.

A useful vector to obtain expression consists of pBR322 sequences which provides a selectable marker for selection in E. coli (ampicillin resistance) as well as an E. coli origin of DNA replication. These sequences are derivable from the plasmid pML-1. The SV40 origin is derivable from a 342 base pair PvuII-HindIII fragment encompassing this region (35, 36) (both ends being convertable to EcoRI ends). These sequences, in addition to comprising the viral origin of DNA replication, encode the promoter for both the early and late transcriptional unit. The orientation of the SV40 origin region is such that the promoter for the late transcriptional unit is positioned proximal to the gene encoding interferon.

### Brief Description of the Drawings

Figure A is a schematic diagram of urokinase polypeptides. Low molecular weight urokinase begins at amino acid 136 and ends at amino acid 412. High molecular weight urokinase starts at amino acid 1 and terminates at amino acid 412. Conversion of the one chain form of both high and low molecular weight urokinase to the bioactive two-chain form occurs by proteolytic cleavage between amino acid 158 and 159. Pre-urokinase begins at amino acid-20. The depicted conformational positioning of the disulfide bonds is based on analogy with other serine proteases.

Figures 1A to 1C depict the nucleotide sequence and restriction endonuclease map of the plasmid pD2 cDNA insert for low molecular weight 33000 dalton urokinase bioactive protein. The nucleotide portion of the synthetic deoxyoligonucleotide CB6B probe is underlined.

Figures 2A,B depict the deduced amino acid sequence of the cDNA sequence of Figure 1, the amino acids of the cDNA insert portion being numbered 1 to 279.

Figures 3A to 3C depict the nucleotide sequence and restriction endonuclease map of the cDNA for full length human urokinase protein. The CB6B probe is likewise underlined.

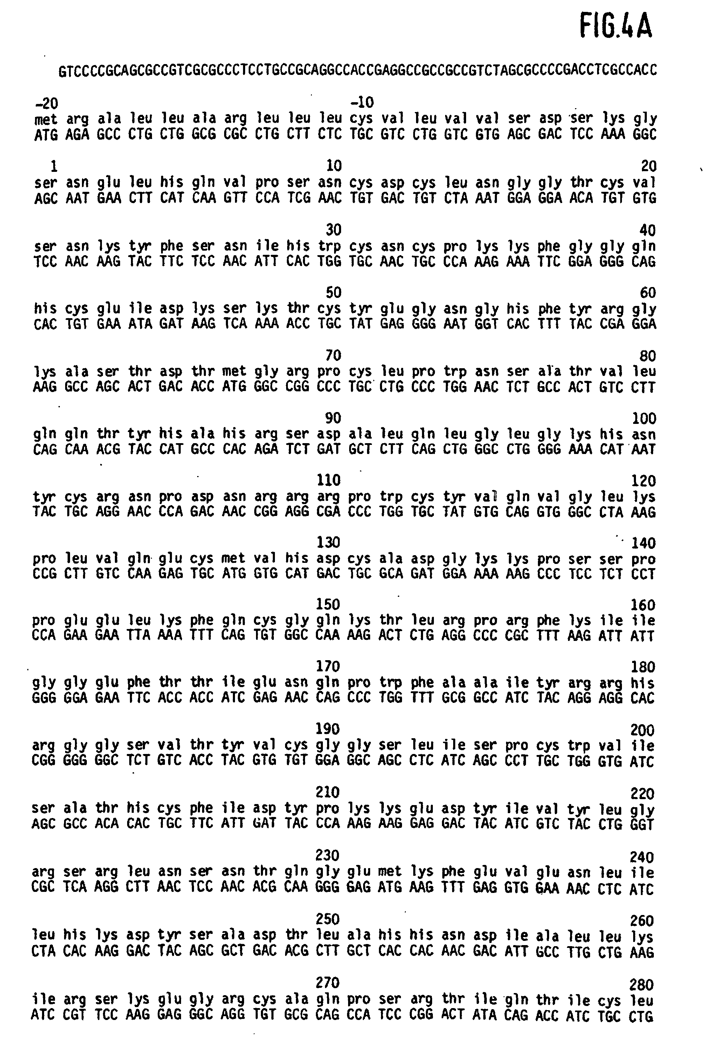

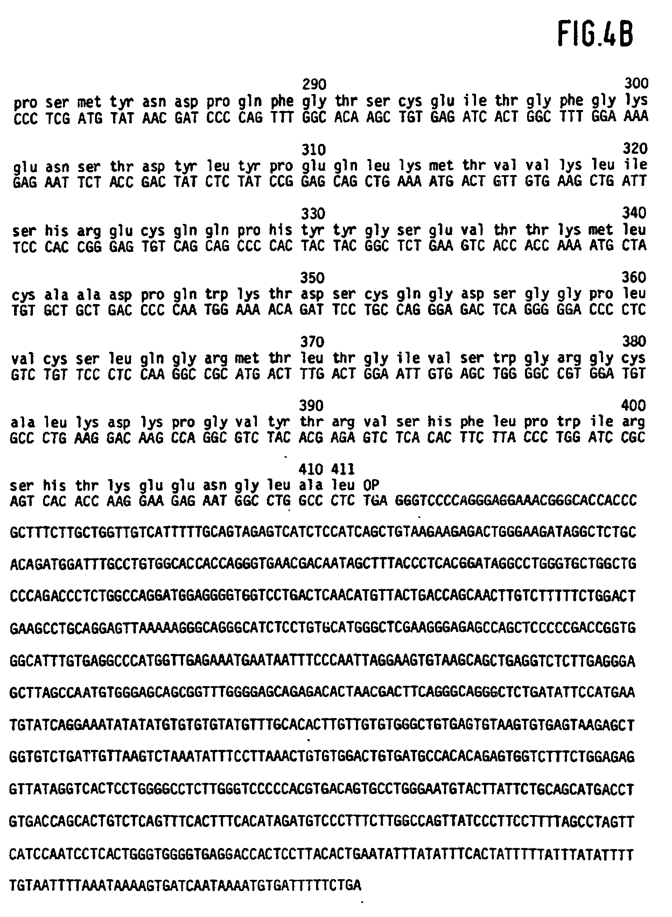

Figures 4A,B depict the deduced amino acid sequence for full length urokinase from the cDNA sequence of Figure 3.

Figure 5 illustrates the construction of plasmid pUK33trpLE_{L} for expression of long fusion -33000 dalton protein.

Figure 6 illustrates the construction of plasmid pUK33trpLE_{S} for expression of short fusion -33000 dalton protein.

Figure 7 shows the construction of the plasmid for direct expression of the 33000 dalton protein.

Figure 8 illustrates another construction of a plasmid for direct expression of the 33K dalton protein.

Figures 9 and 10 illustrate the construction of plasmids for the direct expression of 54K urokinse and a precursor form of 54K urokinase.

Figure 11 shows the construction of a plasmid (p-pEH3-Bal14 preUK54) for the expression of 54K urokinase in eukaryotic cells.

Figure 12 illustrates the time dependent activation of, and the requirement for, plasminogen in a plasmin assay by urokinase produced as described herein.

Figure 13 illustrates the plasminogen activating activity of the urokinase extracts hereof and the inhibition of that activity by antibodies raised against natural urokinase.

### Detailed Description

### A. Source of Urokinase mRNA

Detroit 562 (human pharangeal carcinoma) cells (38)(ATCC No. CCL 138) were cultured to confluency in Eagle's minimal essential medium (39) supplemented to contain 3 percent sodium bicarbonate (pH 7.5), 1 percent L-glutamine (Irvine), 10 percent fetal bovine serum, 1 percent sodium pyruvate (Irvine), 1 percent non-essential amino acids (Irvine), 2.4 percent HEPES (pH 7.5), 50 µg/ml Garamycin, and incubated at 37°C in a 5 percent CO₂ atmosphere. Confluent cells were harvested by centrifugation after treatment with 0.25 percent trypsin for 15 minutes at 37°C.

### B. Messenger RNA Isolation

Total RNA from Detroit 562 cells was extracted essentially as reported by Lynch et al. (40). Cells were pelleted by centrifugation and approximately one gram of cells was then resuspended in 10 ml of 10 mM NaCl, 10 mM Tris^{·}HCl (pH 7.4), 1.5 mM MgCl₂. Cells were lysed by the addition of non-ionic detergent NP-40 to a final concentration of 1 percent. Nuclei were removed by centrifugation and the RNA was further purified by two successive phenol (redistilled)/chloroform: isoamyl alcohol extractions at 4°C. The aqueous phase was made 0.2 M NaCl and total RNA was precipitated by addition of two volumes of 100 percent ethanol and overnight storage at -20°C. Following centrifugation polyA mRNA was purified from total RNA by oligo-dT cellulose chromatography (41). Yields from 1 gram of cells were typically 10-15 mg of total RNA and ∼2 percent of that was polyA mRNA.

### C. Size Fractionation of polyA mRNA

Size fractionation of 200 µg polyA mRNA was achieved by electrophoresis through an acid-urea gel composed of 1.75 percent agarose, 25 mM sodium citrate (pH 3.8) and 6 M urea (40,42). Electrophoresis was performed for 7 hours at 25 mA and 4°C. The gel was then fractionated manually with a razor blade. Individual slices of the gel were melted at 70°C, diluted into 12 mls 10 mM NaCl, 10 mM Tris ^{·} HCl (pH 7.4), 1.5 mM MgCl₂, 0.1 percent SDS and extracted twice with water saturated, redistilled phenol and once with chloroform. Fractions were then ethanol precipitated and subsequently translated in vitro (43) in order to determine the affected size fractionation and integrity of the polyA mRNA.

### D. Preparation of Oligo-dT Primed Colony Library Containing Urokinase DNA Sequences

Poly A mRNA was size-fractionated on acid urea gels. mRNA fractions greater than 12S were pooled and used as template for oligo-dT primed preparation of double stranded cDNA by standard procedures (44,45). The cDNA was size fractionated on 6 percent polyacrylamide gel electrophoresis and 132 ng of ds cDNA greater than 350 basepairs in length was obtained by electroelution. A 30 ng portion of ds cDNA was extended with deoxy C residues using terminal deoxynucleotidyl transferase (46) and annealed with 200 ng of the plasmid pBR322 (37) which had been similarly tailed with deoxy G residues at PstI site (46). Each annealed mixture was then transformed into E. coli K12 strain 294 (ATCC No. 31446). Approximately 10000 ampicillin sensitive, tetracycline resistant transformants were obtained.

### E. Preparation of Synthetic DNA Oligomers for Use as Urokinase Screening Probes

Eight synthetic DNA oligomers 14 bases long were designed complementary to mRNA based on the Met-Tyr-Asn-Asp-Pro amino acid sequence of a urokinase cyanogen bromide polypeptide fragment designated CB6. These eight deoxyoligonucleotides were synthesized by the phosphotriester method (17) in the following pools of two: (CB6A) 5' GGGTCGTTA/GTACAT 3', (CB6B) 5' GGATCGTTA/GTACAT 3', (CB6C) 5' GGGTCATTA/GTACAT 3', (CB6D) 5' GGATCATTA/GTACAT 3'. Each pool of two oligomers was then radioactively phosphorylated as follows: 250 ng of deoxyoligonucleotide were combined in 25 µl of 60 mM Tris^{·}HCl (pH 8), 10 mM MgCl₂, 15 mM beta-mercaptoethanol, and 100 µCi (γ-³²P) ATP (Amersham, 5000 Ci/mMole). 5 units of T4 polynucleotide kinase were added and the reaction was allowed to proceed at 37°C for 30 minutes and terminated by addition of EDTA to 20 mM.

### F. Screening of Oligo dT Primed Colony Library for Urokinase Sequences

∼10000 colonies were individually inoculated into wells of microtitre dishes containing LB (48) + 5 µg/ml tetracycline and stored at -20°C after addition of DMSO to 7 percent. Individual colonies from this library were transferred to Schleicher and Schuell BA85/20 nitrocellulose filters and grown on agar plates containing LB + 5 µg/ml tetracycline. After ~10 hours growth at 37°C the colony filters were transferred to agar plates containing LB + 5 µg/ml tetracycline and 12.5 µg/ml chloramphenicol and reincubated overnight at 37°C. The DNA from each colony was then denatured and fixed to the filter by a modification of the Grunstein-Hogness procedure (49). Each filter was floated for 3 minutes on 0.5 N NaOH, 1.5 M NaCl to lyse the colonies and denature the DNA then neutralized by floating for 15' on 3 M NaCl, 0.5 M Tris^{·}HCl (pH 7.5). The filters were then floated for an additional 15 minutes on 2XSSC, and subsequently baked for 2 hours in an 80°C vacuum oven. The filters were prehybridized for ∼2 hours at room temperature in 0.9 M NaCl, 1X Denhardts, 100 mM Tris^{·}HCl (pH 7.5), 5 mM Na-EDTA, 1 mM ATP, 1 M sodium phosphate (dibasic), 1 mM sodium pyrophosphate, 0.5 percent NP-40, and 200 µg/ml E. coli t-RNA, and hybridized in the same solution overnight essentially as described by Wallace et al. (50) using ∼40x10⁶ cpm of each kinased CB6 deoxyoligonucleotide pool of 2. After extensive washing at 37°C in 6X SSC, 0.1 percent SDS, the filters were exposed to Kodak XR-5 X-ray film with DuPont Lightning-Plus intensifying screens for 16-24 hours at -80°C. Two colonies indicated hybridization with the mixture of eight probes: UK513dT69D2 (pD2) and UK513dT73D12 (pD12).

### G. Characterization of pD2 and pD12 Plasmid DNA

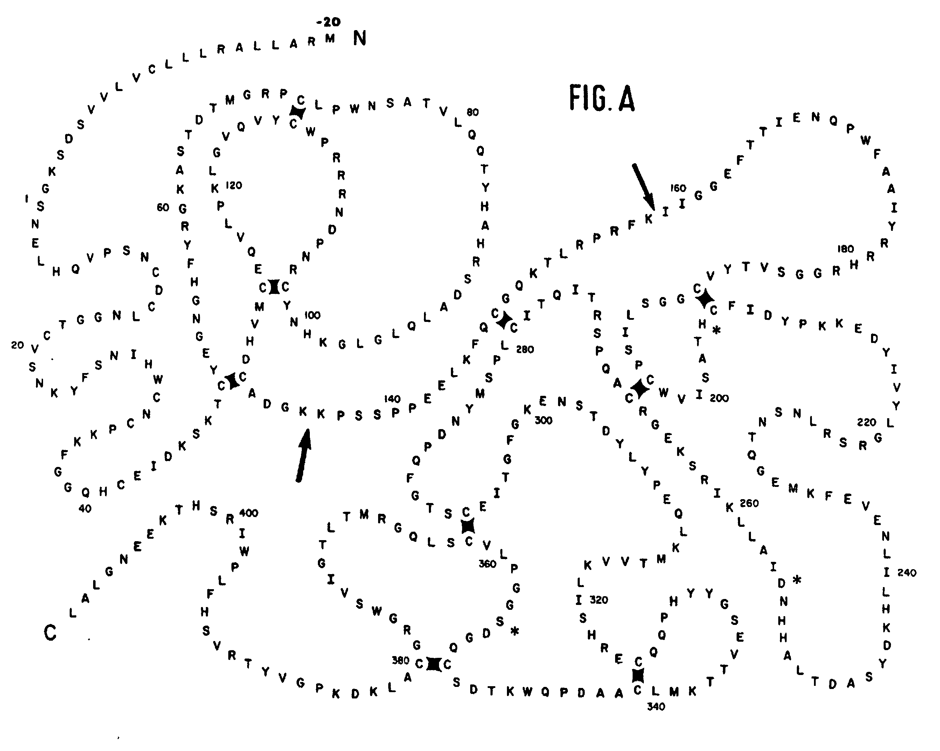

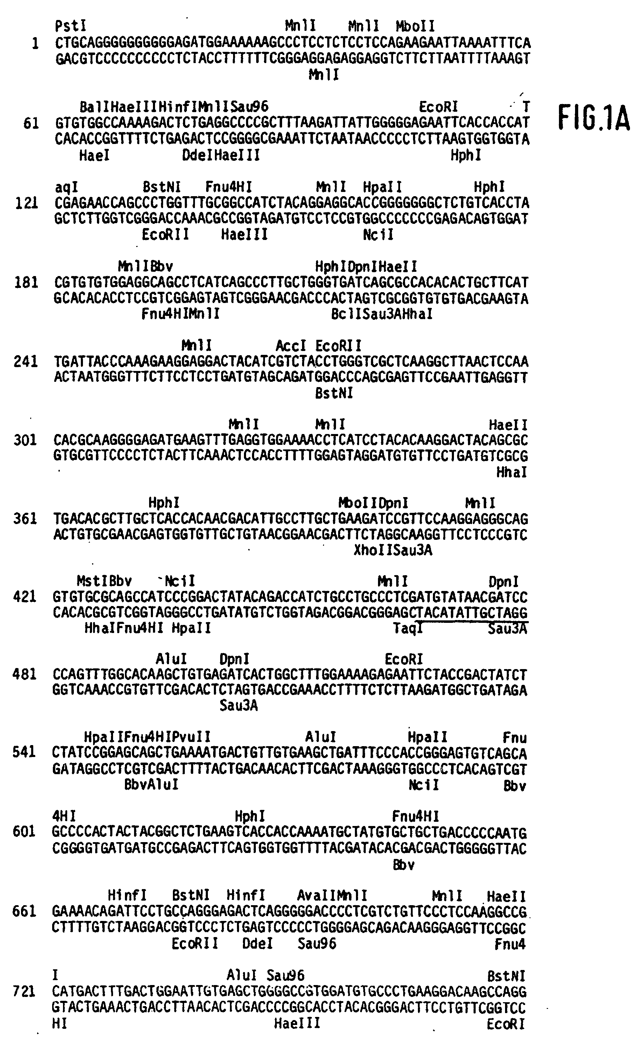

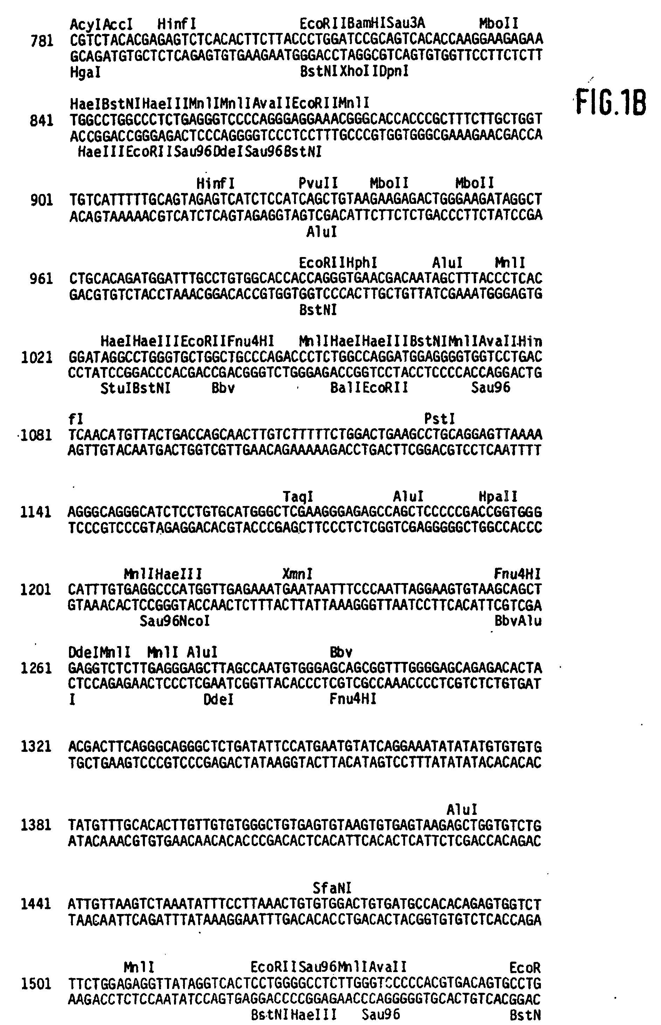

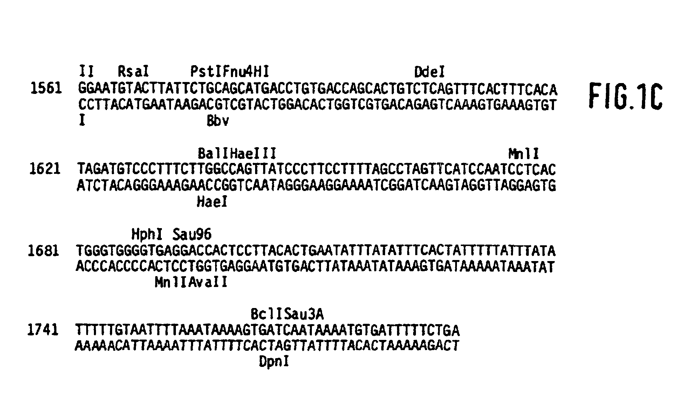

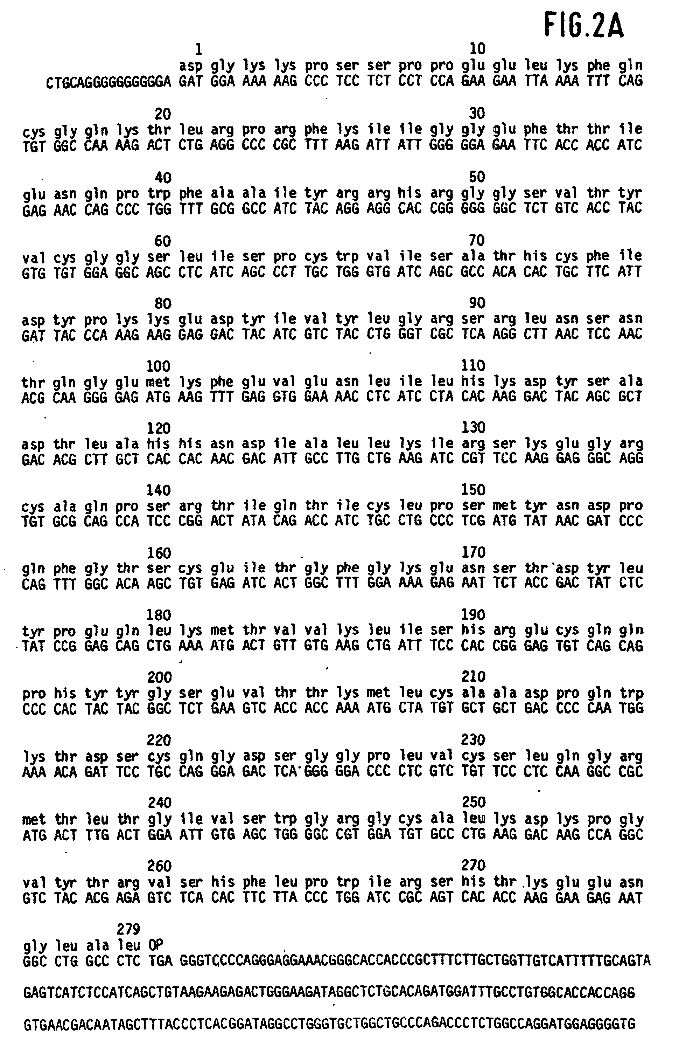

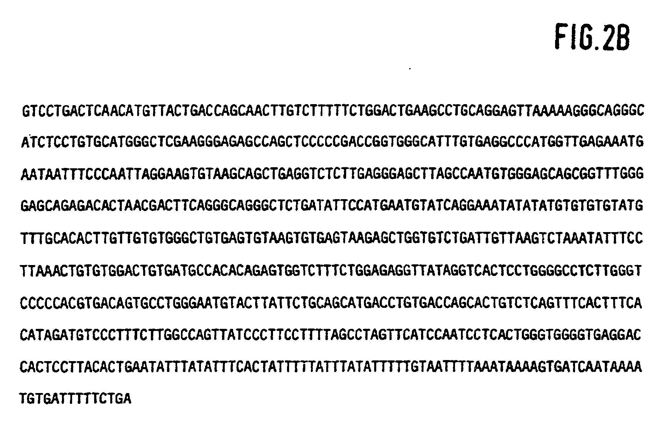

Plasmid DNA isolated from E. coli colony UK513dT69D2 and UK513dT73D12 by a rapid miniscreen method (51) was subjected to PstI restriction endonuclease analysis. This analysis strongly suggested that pD2 and pD12 are identical. Each plasmid DNA has 3 PstI restriction fragments that comigrate when electrophoresed through a 6 percent polyacrylamide gel. The complete nucleotide sequence of the plasmid pD2 cDNA insert was determined by the dideoxynucleotide chain termination method (52) after subcloning the PstI restriction fragments into the M13 vector mp7 (53). Figure 1 presents the nucleotide sequence and Figure 2 presents the translated nucleotide sequence of the cDNA insert fragment of pD2. The entire coding region of low molecular weight (33K) urokinase was isolated on this one large fragment of pD2. The nucleotide sequence of the CB6B (5' GGATCGTTA/GTACAT) deoxyoligonucleotide includes nucleotides 466 through 479 according to this map. A typical serine protease active site (gly asp ser gly gly pro) is present between amino acids 222 and 227. The coding region consists of 838 basepairs or 279 amino acids of the carboxy terminal portion of high molecular weight (54K) urokinase. The stop codon UGA at amino acid position 280 begins ca. 935 nucleotides of 3' untranslated sequence up to the poly A sequence. Because only 31413 daltons of full length urokinase were encoded by the cDNA insert of pD2 it was necessary to construct additional colony banks containing urokinase sequences in order to identify high molecular weight urokinase.

### H. Construction of Two Different Specifically Primed Colony Banks for Amino Terminal Extension of the Existing Urokinase Clone

The first specifically primed cDNA bank utilized a 45 basepair urokinase DNA restriction endonuclease fragment beginning with HaeII in position 225 and ending with AccI in position 270 (Figure 1) as a primer rather than oligo dT₁₂₋₁₈. This fragment was heat denatured in the presence of 20 µg unfractionated Detroit 562 polyA mRNA and cDNA was prepared according to procedures referenced in Section D. 11.5 ng of double-stranded cDNA greater than 200 bp were electroeluted from a 6 percent polyacrylamide gel, and used to generate approximately 6000 clones in E. coli 294.

A second specifically primed cDNA bank called UK89CB6 of about 4000 colonies was constructed using a pool of 4 µg polyA mRNA acid-urea agarose gel fraction 8 and 4 µg polyA mRNA from fraction 9 (Section C). 250 ng of each CB6 deoxyoligo- nucleotide pool (Section E) were used as primer rather than oligo dT₁₂₋₁₈.

### I. Screening of Full Length Colony Bank

Full length cDNA containing colonies were transferred directly to nitrocellulose filters then grown at 37°C. The colonies were lysed and the DNA was denatured and fixed to the filters as described in Section F (49). A ³²P-labelled DNA probe was prepared (54) from a 143 basepair HinfI to HaeII restriction endonuclease fragment from the cDNA insert of pD2 and hybridized (55) with the filter fixed full length cDNA. 8x10⁶ CPM of the probe was hybridized for 16 hours then washed as described (55) and exposed to X-ray film. Two colonies demonstrated strong hybridization: A3 and E9.

### J. Characterization of Full Length Urokinase cDNA's pA3 and pE9

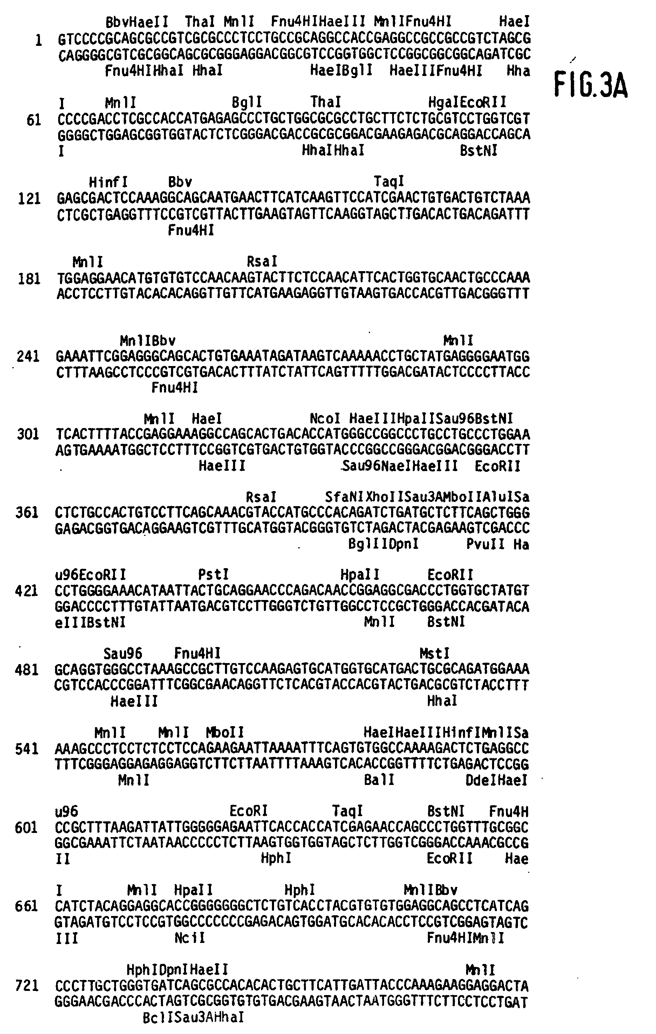

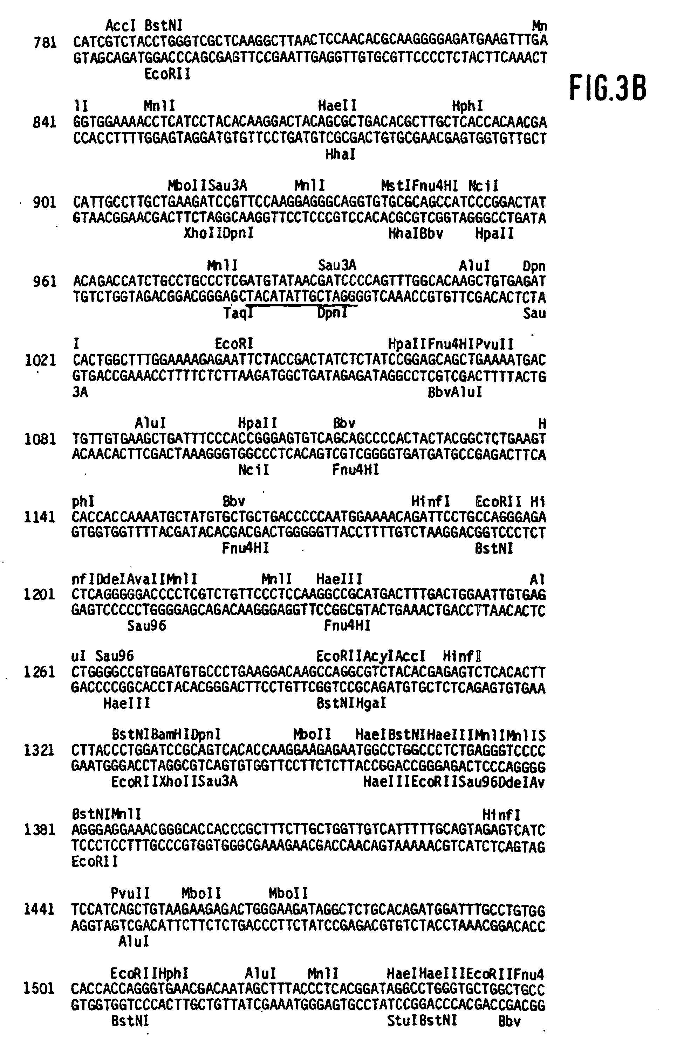

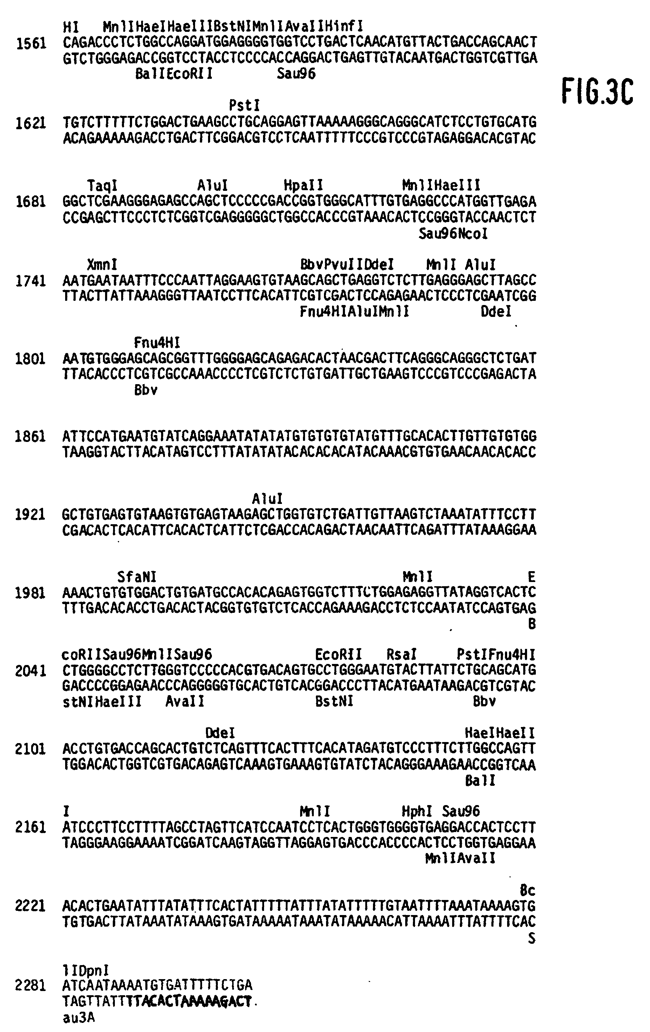

PstI restriction analysis of A3 plasmid DNA showed cDNA insert fragments of ∼360 bp and ∼50 bp, and of E9 plasmid DNA, one fragment at ∼340 bp. PstI EcoRI double restriction of each plasmid DNA revealed a common cDNA insert fragment of ∼190 bp as predicted where each plasmid DNA encoded urokinase sequence information 5' to the HaeII AccI primer fragment. Plasmid pA3 has an additional PstI EcoRI cDNA insert fragment of 185 bp and E9 a 160 bp additional fragment. The larger ∼360 bp PstI cDNA insert fragment of pA3 was subcloned into the M13 vector mp7 (53) and sequenced by the dideoxynucleotide chain termination method (52). The urokinase coding sequence of pA3 is located from approximately position 405 to position 785 in the cDNA sequence for full length urokinase protine in Figure 3.

### K. Screening of Urokinase Colony Bank UK89CB6

DNA from ∼1900 UK89CB6 cDNA insert containing colonies was denatured and fixed to nitrocellulose filters as previously described in Section F. A ³²P-labelled DNA probe was prepared (54) from the 146 bp PstI HinfI fragment of the cDNA insert fragment of pA3. 40x10⁶ CPm of this probe was then hybridized to the filter bound DNA of UK89CB6 colonies for 16 hours then washed as described (55) and exposed to X-ray film. Two colonies demonstrating positive hybridization were UK89CB6F1 (pF1) and UK89CB6H10 (pH10).

### L. Characterization of pFI and pH10 Urokinase cDNAs

PstI restriction of pF1 demonstrates cDNA insert fragments of ∼450 bp and ∼125 bp, and pH10 shows one PstI cDNA insert fragment of ∼500 bp. PstI EcoRI double restriction of pF1 indicates no EcoRI restriction site and has CDNA insert fragments identical to those of the PstI restriction alone. pH10 does demonstrate an EcoRI restriction site, producing cDNA insert fragments of ∼375 bp and ∼110 bp. This EcoRI site of pH10 is probably the same EcoRI site as noted at position 627 (Figure 3). The pF1 cDNA insert does not contain this EcoRI restriction site.

pF1, having a cDNA insert fragment longer than that of pH10, was selected for sequencing. Both PstI restriction fragments of the pF1 cDNA insert were sequenced by M13 subcloning and dideoxy sequencing. The composite nucleotide sequence of UK cDNA inserts from pF1, pA3 and pD2 encoding the entire amino acid sequence of high molecular weight full length urokinase is shown in Figure 3. The urokinase coding sequence of pF1 is depicted from position 1 approximately to position 570. The urokinase coding sequence of pD2 is located from position 532 to position 2304 in Figure 3.

The amino terminal serine at amino acid position one as determined by amino acid sequence analysis is shown in Figures A and 4. The preceding 20 amino acids at the amino terminus beginning with met and ending with gly probably serves as a signal sequence for the secretion of the remaining 411 amino acids of high molecular weight urokinase. This putative signal sequence has features, such as size and hydrophobicity (56,57), in common with other characterized signal sequences.

Trypsin cleavage sites rendering 33K two-chain low molecular weight urokinase from high molecular weight urokinase are as follows: lys at position 136 is the amino terminal amino acid of the short chain and ile at position 159 is the amino terminus of the long chain (Figures A, 4).

### M. Expression of Low Molecular Weight Derivatives of Urokinase in E. coli

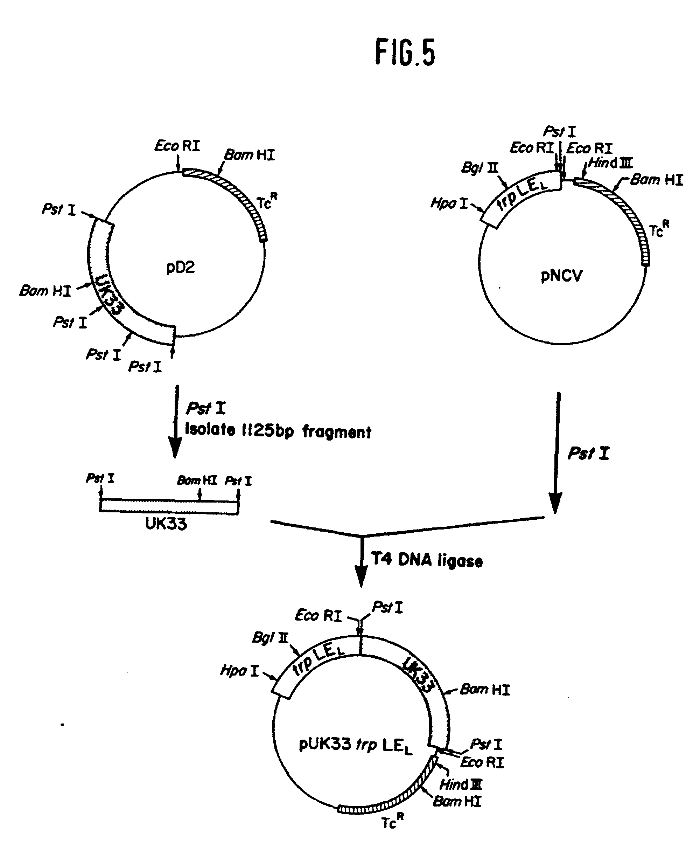

### 1. Long trp LE fusion (Figure 5)

A plasmid (pNCV, 58) was constructed which has the following properties: 1) the plasmid is a derivative of pBR322 and is present in about 20 copies per cell. 2) the plasmid makes its E. coli host resistant to tetracycline. 3) the plasmid contains an inducible tryptophan promoter, which directs the synthesis of a protein consisting of a fusion between the trp leader peptide and the trp E structural gene (LE fusion gene). 4) A unique PstI restriction site was constructed in the trp E gene, which can be used to clone PstI DNA fragments, by converting the EcoRI site at the distal end of the LE gene in plasmid pSOM7Δ1Δ4 to a PstI site flanked by two EcoRI sites using a synthetic sequence: 
AATTCTGCAG
    GACGTCTTAA.
 The DNA fragment containing the trp promoter and LE gene was then introduced into plasmid pBR322 to give plasmid pNCV (47A).

The urokinase PstI fragment from nucleotide position 5 (the Pst I 5'- cleavage site) to nucleotide position 1130 (Figure 1) was cloned into the PstI site of pNCV in such a way that a fusion protein is made upon induction of the trp promoter. The N-terminal part is trp LE and the C-terminal part is low molecular weight urokinase.

With reference to Figure 5, 5 µg of plasmid pUK513dT69D2 (pD2) was digested with 20 units PstI and the 1125 bp cDNA insert fragment encoding low molecular weight urokinase was isolated by 6 percent polyacrylamide gel electrophoresis. ~1 µg of this insert was electroeluted from the gel, phenol/chloroform extracted and ethanol precipitated. 1 µg of the vector plasmid pNCV (58) was digested with 10 units PstI and ethanol precipitated after phenol/chloroform extraction. -100 ng of this 1125 bp fragment was combined with ^{~}100 ng PstI digested pNCV in 20 µl of 20 mM Tris^{·}HCl (pH 7.5), 10 mM MgCl₂, 10 mM DTT, 2.5 mM ATP and 30 units of T4 DNA ligase. After overnight ligation at 14°C, one half of the mixture was transformed in E. coli K12 strain 294. BamHI digestion of the DNA from twelve transformants showed 3 with the proper orientation. Expression of this plasmid (pUK33trpLE_{L}) (Figure 5) in E. coli yielded a long trp LE fusion protein including 33000 urokinase. The 33000 urokinase is activated by cleavage with a trypsin-like active enzyme between positions 3 and 4 and positions 26 and 27 (see Figure 2).

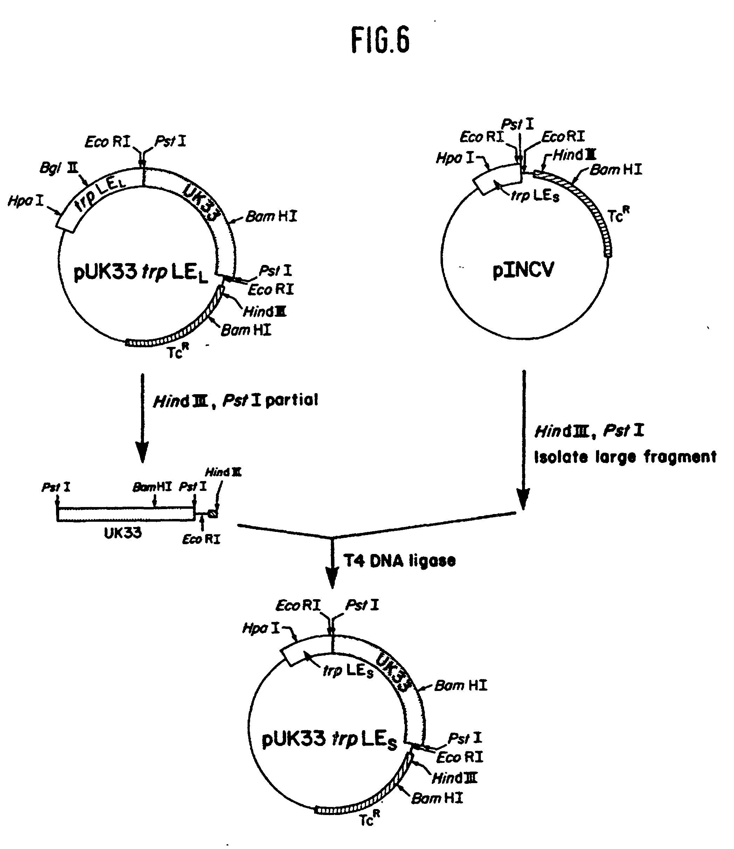

### 2. Short trp LE fusion (Figure 6)

Plasmid pINCV was constructed. It is similar to pNCV (see supra.) in every respect except that the majority of the trp E gene is deleted. In this plasmid the BglII site was converted to a PstI site and the region between this new PstI site and the original PstI site was deleted. ∼100 ng of pINCV was digested with PstI and HindIII then phenol/CHCl₃ extracted and ethanol precipitated. ∼3 µg of pUK33trpLE_{L} (Figure 6) was digested to completion with HindIII and partially digested with PstI to yield a PstI HindIII DNA fragment of 1150 bp which was purified by electroelution after electrophoresis on a 6 percent polyacrylamide gel. The PstI site within the structural UK gene was spared from digestion. All of the PstI HindIII digested pINCV was mixed with ∼50 ng of the 1150 bp HindIII, partial PstI fragment of pUK33trpLE_{L} and ligated overnight at 14°C. This mixture was then transformed into E. coli K12 strain 294. BamHI digestion confirmed the proper construction of this plasmid (pUK33trpLE_{S}) (Figure 6). Expression of this plasmid in E. coli yielded a fusion protein from which 33,000 urokinase is activated, as described supra.

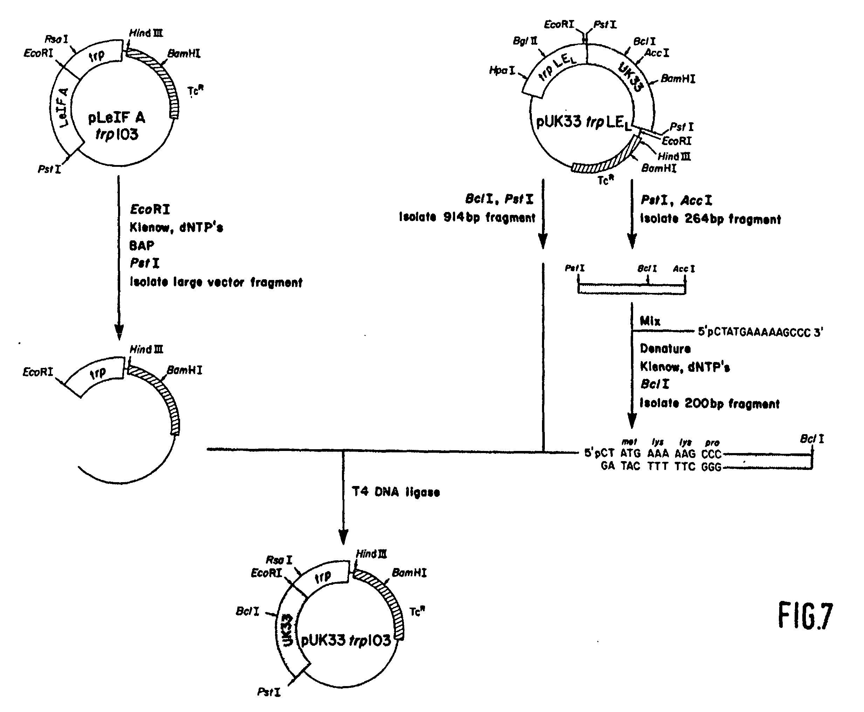

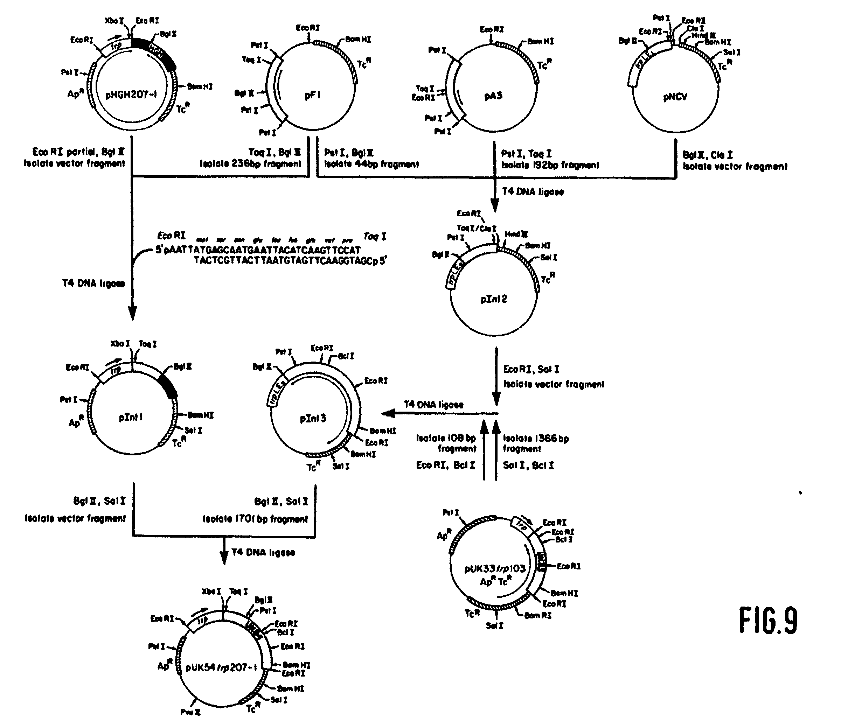

### 3. Direct Expression of 33K Urokinase (Figures 7 and 9)

A urokinase DNA fragment beginning with nucleotide 16 (Figure 1), was cloned into a pBR322 derivative resulting in a construction in which the trp promoter is positioned directly in front of this urokinase fragment encoding low molecular weight urokinase. The plasmid pLeIFAtrp103 (Figure 7) is a derivative of the plasmid pLeIFA25 (58) in which the Eco RI site distal to the LeIFA gene has been removed (59). 10 µg of pLeIFAtrp103 (Figure 7) was digested with 20 units EcoRI phenol/CHCl₃ extracted and ethanol precipitated. The EcoRI cohesive ends of the plasmid DNA molecules were extended to flush ends using 12 units of DNA Polymerase I in a 50 µl reaction containing 60 mM NaCl, 7 mM MgCL₂, 7 mM Tris^{·}HCl (pH 7.4) and 1 mM in each ribonucleotide triphosphate. The reaction was incubated at 37°C for 1 hour, extracted with phenol/CHCl₃ and precipitated with ethanol. The DNA was then resuspended in 50 µl of 10 mM Tris^{·}HCl (pH 8), 1 mM EDTA and treated with 500 units Bacterial Alkaline Phosphatase for 30 minutes at 65°C, twice extracted and ethanol precipitated. After digestion with PstI the mixture was electrophoresed on a 6 percent polyacrylamide gel and the ∼3900 bp vector fragment was electroeluted.

The plasmid pUK33trpLE_{L} was transformed into E. coli K12 strain GM48 (deoxyadenosine methylase⁻) in order that DNA purified from this E. coli strain could be digested with restriction endonuclease BclI (60). 4 µg of this DNA were treated for 1 hour at 50°C with 6 units of BclI (in 75 mM KCl, 6 mM Tris^{·}HCl (pH 7.4), 10 mM MgCl₂, 1 mM DTT), then made 50 mM NaCl and digested with 10 units PstI. 6 percent gel electrophoresis was performed and the 914 bp fragment was electroeluted.

A 14 nucleotide DNA primer encoding the amino acid sequence met lys lys pro was synthesized by the phosphotriester method (47) and has the following sequence: 
MetLysLysPro
5' CTATGAAAAAGCCC 3'
 500 ng of this primer were phosphorylated at the 5' end with 10 units T4 DNA Kinase in a 20 µl reaction containing 0.5 mM ATP. The 264 bp PstI AccI cDNA insert fragment of pUK33trpLE_{L} (grown in E. coli GM48) was isolated. -500 ng of this fragment resuspended in 10 µl of deionized water were mixed with the 20 µl of the phosphorylated primer, heated to 95°C for 3 minutes and quick frozen in a dry-ice ethanol bath. The denatured DNA solution was made 60 mM NaCl, 7 mM MgCl₂, 7 mM Tris^{·}HCl (pH 7.4), 1 mM in each deoxy ribonucleotide triphosphate and 12 units DNA polymerase I large fragment was added. After 2 hours incubation at 37°C this primer repair reaction was phenol/CHCl₃ extracted and ethanol precipitated and digested to completion with BclI at 50°C. The reaction mixture was then run on a 6 percent polyacrylamide gel and ∼50 ng of the 200 bp amino-terminal blunt-end to BclI fragment was electroeluted. Subsequently, ∼50 ng of the blunt-BclI primer-repair fragment, ∼100 ng of the BcII PstI carboxy-terminal fragment and ∼100 ng of the ∼3900 bp vector fragment were ligated overnight at 14°C and transformed into E. coli 294. EcoRI digestion of a number of transformants indicated the proper construction and DNA sequence analysis proved the desired sequence through the initiation codon of this new plasmid, pUK33trp103 (Figure 7). In this construction, the N-terminal methionine is followed by two lysines which in turn are followed by the amino acid sequence 5 through 279 as depicted in Figure 2A. Expression of this plasmid in E. coli resulted in the synthesis of low molecular weight urokinase. This protein was activated with a trypsin-like active enzyme as described supra which serves to cleave the N-terminal lysine pair and cleaves between lysine in position 26 and isoleucine in position 27 (Figure 2A).

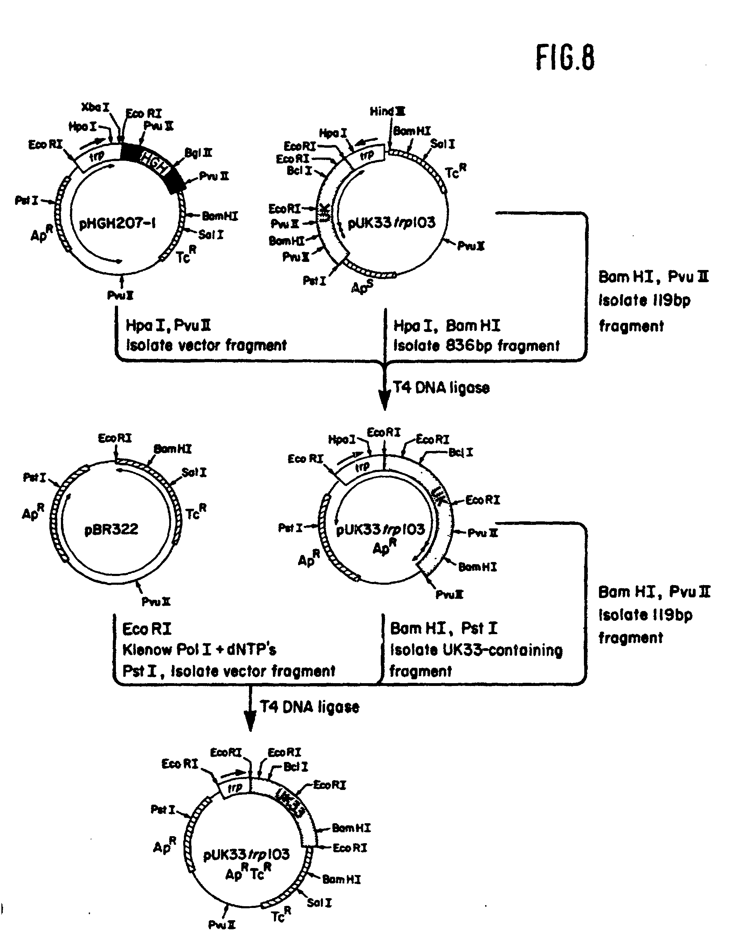

We found it desirable to construct a derivative plasmid of pUK33trp103 that would confer tetracycline resistance to its host cell. Figure 8 depicts the following construction of pUK33trp103Ap^{R-}Tc^{R}. 5µg of pHGH207-1 (See infra) was digested with HpaI and PvuII. The vector fragment was isolated and purified. 5µg of pUK33trp103 was digested with Hpal and BamH1, electrophoresed on 6 percent polyacrylamide and the 836 bp DNA fragment was purified. A second 5µg aliquot of pUK33trp103 was digested with BamH1 and PvuII for isolation and purification of the 119 bp DNA fragment. Equal molar amounts of each of these three DNA fragments were ligated overnight at 14°C and used to transform E. coli 294. Restriction endonuclease analysis of plasmid DNA from several ampicillin resistant transformants verified the proper construction of pUK33trp103Ap^{R} and the reversal in orientation of the trp promoter/UK33 encoding DNA.

∼5µg pBR322 DNA was digested with EcoR1 and the cohesive ends were filled in with Klenow Poll. After Pst1 digestion, the large vector fragment containing the DNA that encodes tetracycline resistance, the origin of replication, and a portion of the ampicillin resistance gene was isolated and purified. ∼5µg of pUK33trp103 Ap^{R} was digested with BamH1 and Pst1. The DNA fragment encoding the remaining portion of the ampicillin resistance gene, the trp promoter and most of low molecular weight urokinase was purified. Approximately equal molar amounts of these two DNA fragments and the 119 bp BamH1-PvuII DNA fragment from pUK33trp103Ap^{R} was ligated overnight at 14°C to complete the construction of pUK33trp103Ap^{R}Tc^{R}. This plasmid was employed in the construction of a plasmid designed to express high molecular weight full length urokinase (see Section N and Figure 9).

### N. Expression of High Molecular Weight Derivatives of Urokinase

### 1. Direct Expression of 54K Urokinase

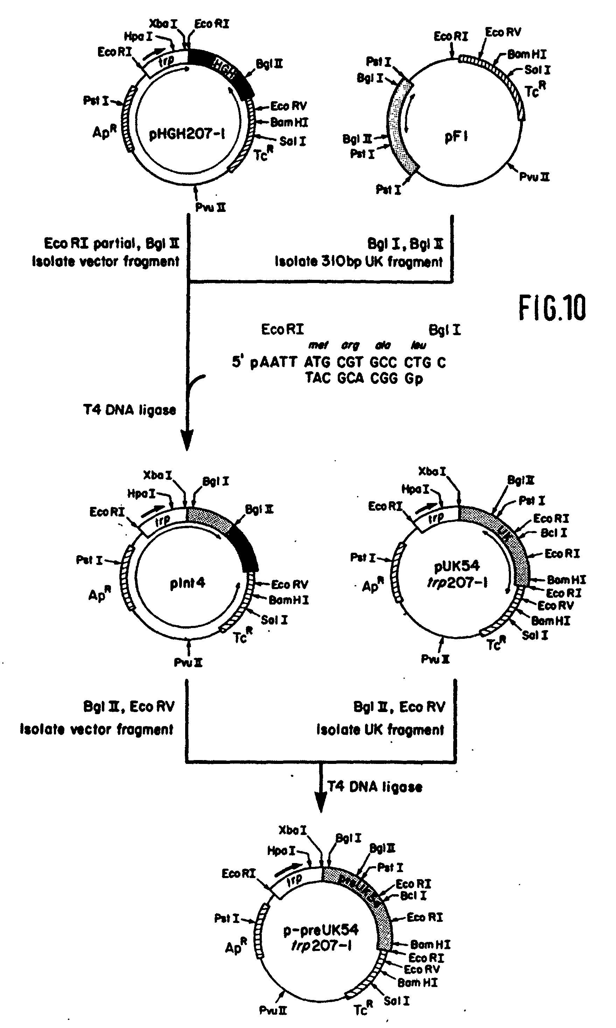

Figure 10 illustrates construct for full length urokinase. Plasmid pHGH207-1, having a single trp-promoter, was obtained by removal of the double lac-promoter from pHGH 207 that has a double lac-promoter followed by a single trp-promoter. This was done as follows: The trp-promoter 310bp DNA fragment was obtained from pFIF trp 69 (20) by digestion with EcoRI. This fragment was inserted into pHGH 107 (44) that had been opened with EcoRI. Thus, a plasmid was obtained (pHGH 207) that has a double lac promoter followed by the trp-promoter, flanked by EcoRI sites. The thus obtained pHGH 207 was digested with BamHI; this was partially digested with EcoRI and the largest fragment was isolated. This fragment therefore has the entire trp-promoter. From pBR322 the largest EcoRI-BamHI fragment was isolated. Both fragments were ligated and the mixture was used to transform E. coli 294. Tetr, Amp^{r} colonies were isolated and most of them had the plasmid with the structure as shown for pHGH207-1. Plasmid pHGH207-1 is thus a derivative of the plasmid pHGH107 (44) and has the following properties: 1) the human growth hormone gene is flanked by the tryptophan promoter rather than the lac promoter as with pHGH107, 2) the plasmid confers ampicillin and tetracycline resistance when expressed in E. coli. (See also 47A).

20 µg of pHGH207-1 was partially digested with EcoRI and totally digested with BglII. Purification of the large vector fragment was achieved by 5 percent polyacrylamide gel electrophoresis, electroelution, phenol/CHCl₃ extraction and ethanol precipitation. 14 µg of pF1 was digested with BglII and TaqI and the 236 bp DNA fragment was isolated and purified from a 6 percent polyacrylamide gel. The following complementary DNA fragments were synthesized by the phosphotriester method (47): 
MetSerAsnGluLeuHisGlnValPro
5' AATTATGAGCAATGAATTACATCAAGTTCCAT
TACTCGTTACTTAATGTAGTTCAAGGTAGC 5'
 As indicated, the amino acid sequence Met Ser Asn Glu Leu His Gln Val Pro encodes the initiation codon, ATG, and the eight amino-terminal amino acids of high molecular weight urokinase. 50 ng of each synthetic DNA fragment were phosphorylated and the fragments were mixed, heated to 65°C for 1 minute and allowed to cool at room temperature for 5 minutes. 10 ng of the phosphorylated and mixed synthetic DNA fragments were combined with ∼200 ng of the partial EcoRI, BglII pHGH207-1 vector fragment and ∼50 ng of the 236 bp BglII TaqI DNA fragment, ligated overnight at 14°C and transformed into E. coli 294. Individual plasmid DNAs from 24 ampicillin resistant colonies were digested with EcoRI and BglII and one plasmid (pInt1) demonstrating the proper construction was selected for DNA sequence analysis. This analysis verified the correct DNA sequence through the ATG initiation codon and the amino-terminal portion of high molecular weight urokinase.

4 µg of pNCV (Section M) was digested with BglII and ClaI and the large vector fragment was isolated and purified from a 5 percent polyacrylamide gel. 30 µg of pF1 was digested with PstI and BglII and electrophoresed through a 6 percent polyacrylamide gel. The 44 bp DNA fragment was electroeluted, phenol/CHCl₃ extracted and ethanol precipitated. 4 µg of pA3 DNA were digested with PstI and TaqI and the 192 bp DNA fragment was purified from a 6 percent polyacrylamide gel. ∼100 ng of the BglII ClaI vector DNA fragment, ∼50 ng of the 192 bp fragment and ∼50 ng of the 44 bp fragment were combined and ligated overnight at 14°C then transformed into E. coli 294. BglII ClaI double digestion of plasmid DNA from several tetracycline resistant transformants demonstrated the correct construction of this new plasmid named pInt2.

5µg of pUK33trp103Ap^{R}Tc^{R} grown in E. coli GM48 (ATCC No. 39099, April 9, 1982) was digested with Bcl1 and Sal1 for isolation and purification of the 1366 bp DNA fragment. The 108 bp EcoR1-Bcl1 DNA fragment was isolated from the same plasmid. Approximately equal molar amounts of the EcoR1-Sal1 DNA fragment from pUK33trp103Ap^{R}Tc^{R} and the EcoR1-Bcl1 DNA fragment also from pUK33trp103Ap^{R}Tc^{R} were ligated overnight at 14°C to yield pInt3.

5 µg of pInt3 DNA was digested with BglII and SalI, electrophoresed through 6 percent polyacrylamide and the 1701 bp fragment containing the carboxy terminal portion of full length urokinase and the amino terminal portion of the tetracycline resistance gene was purified. ∼5 µg of p intermediate 1 DNA was digested with BglII and SalI, electrophoresed through 6 percent polyacrylamide and the large vector fragment containing the carboxy-terminal portion of the tetracycline resistance gene, the origin of replication, the ampicillin resistance gene, the trp promoter, the initiation codon ATG, and the amino-terminal portion of full length urokinase was purified. ∼100 ng of the vector fragment and ∼100 ng of the 1701 bp fragment were combined, ligated overnight at 14°C and transformed into E. coli 294. A plasmid from a tetracycline resistant and ampicillin resistant colony demonstrating the correct PvuII restriction endonuclease pattern was identified and confirmed the construction of full length urokinase downstream from the trp promoter. This is plasmid pUK54trp207-1. Full length urokinase is produced by expression of this plasmid in E. coli 294.

### 2. Direct Expression of Pre-UK 54K in E. coli (Figure 10)

The following scheme was used to construct a plasmid for direct expression of preUK54. Two complementary DNA fragments were synthesized by the phosphotriester method (47) encoding the amino acid sequence initiation codon ATG followed by the first three N-terminal amino acids of the urokinase presequence Arg Ala Leu as shown below

EcoR1 MetArgAlaLeu Bgl1
5' AATTATGCGTGCCCTGC
        TACGCACGGG5'

EcoR1 and Bgl1 restriction endonuclease cleavage sites flank this portion of the presequence. 50ng of each synthetic DNA fragment was phosphorylated. The phosphorylated fragments were mixed, heated to 65°C for 1 minute and allowed to cool to room temperature. 5µg of pF1 DNA was digested with Bgl1 and Bgl2 and the 310bp UK DNA fragment was isolated and purified. ∼50ng of the 310bp UK DNA fragment, ∼150ng of the partial EcoR1, BglII vector DNA fragment from pHGH207-1 (see Section N) and 10ng of the phosphorylated and mixed synthetic DNA fragments was ligated overnight at 14°C and transformed into E. coli 294. Individual plasmid DNA's isolated from several ampicillin resistant transformants were analyzed to confirm the proper construction and nucleotide sequence of the presequence of high molecular weight urokinase.

One correct plasmid was named pInt4. 5µg of pInt4 DNA was digested with BglII and EcoRv for isolation and purificaton of the vector DNA fragment containing the N-terminal nucleotides of pre-UK54, the trp promoter, ampicillin resistance genes, origin of replication and a portion of the tetracycline resistance encoding DNA. 5µg of pUK54trp207-1 DNA was digested with BglII and EcoRv. The BglIIEcoRv DNA fragment encoding the remainder of UK54 and the tetracycline resistance encoding DNA was isolated, purified and ligated with the BglII EcoRv vector DNA fragment to complete the construction of the plasmid p-preUK54trp207-1.

### O. Direct Expression (Pre-)Urokinase in Tissue Culture

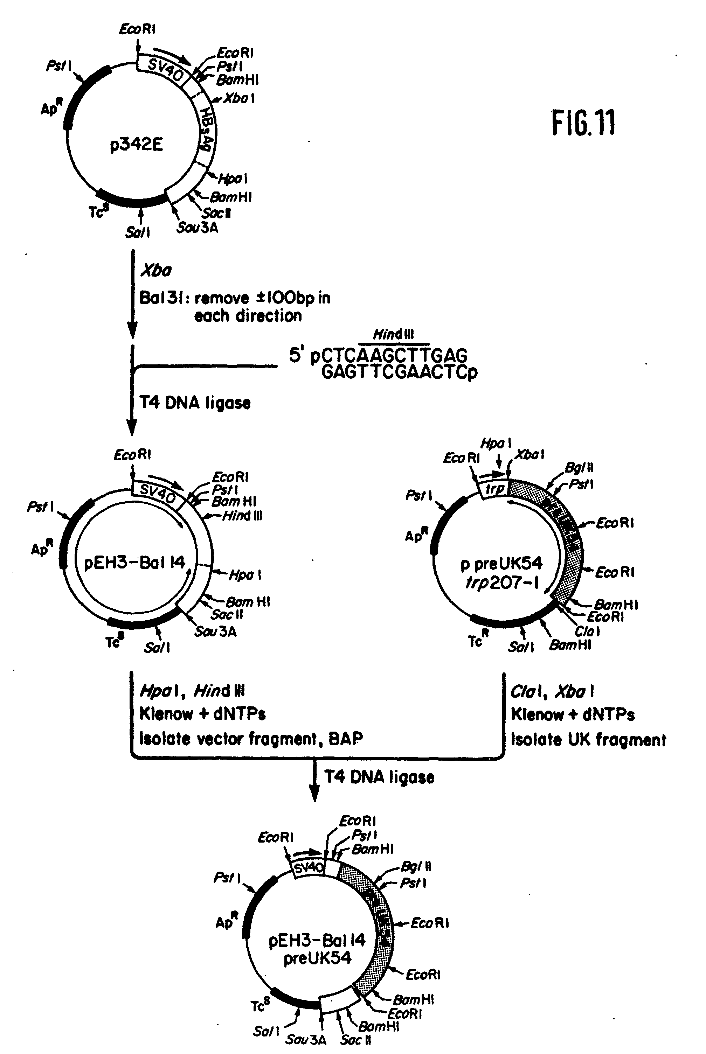

Figure 11 depicts the introductin of the gene encoding preurokinase into the eukaryotic expression vector p342E (62) capable of replication and expression of preurokinase in permissive monkey cells. 10µg of p342E DNA was digested with XBA1 and ∼100 base pairs were removed in each direction using Bal31 nuclease (fragment 1). 100ng of HindIII linker 5' CTCAAGCTTGAG synthesized by the phosphotriester method (47) was phosphorylated, heated to 65°C for 1 minute and allowed to cool to room temperature. The phosphorylated linker and fragment 1 were ligated overnight at 14°C and transformed into E. coli 294. Restriction endonuclease analysis of one transformant named pEH3-Bal14 proved the introduction of a HindIII restriction endonuclease site and the loss of the XBA1 site. 5µg of pEH3-BAL14 DNA was digested with HindIII and Hpa1. The cohesive ends were extended to blunt ends using Klenow Poll. The DNA was treated with BAP and the vector fragment containing the SV-40 early promoter, ampicillin resistance genes and origin of replication was isolated and purified (fragment 2). 5µg of ppreUK54trp207-1 was digested with Cla1 and XBa1. The cohesive ends were extended with Klenow Poll and the DNA fragment encoding preUK54 was isolated and purified (fragment 3). ∼100ng of fragment 2 and ∼100ng of fragment 3 were ligated overnight at 14°C and transformed in E. coli 294. Restriction endonuclease analysis of plasmid DNA named pEH3-BAL14 preUK54 from one transformant verified the correct construction. pEH3-BAL14 pre UK54 DNA was then used to transfect permissive monkey cells (62) for the expression preUK54 and the secretion of full length high molecular weight urokinase.

### P. Isolation and Characterization

Urokinase containing residue was isolated from E. coli. The residue was dissolved in 5M guanidine HCl, containing 50 mM Tris, pH 8.0. The solution was diluted to 1M guanidine HCl, 50mM Tris HCl, pH 9, at a protein concentration of 1 ng/ml. The solution was then brought to 2 mM reduced glutathione (GSH), 0.2 mM oxidized glutathione (GSSG) and incubated overnight at room temperature. The resulting solution containing refolded protein was then dialyzed into aqueous medium. The resulting solution contained urokinase which showed 100PU/mg activity.

Upon purification following conventional techniques, the protein is characterized showing the expected N-terminal sequence for both chains of the low molecular weight, bioactive material. C-terminal analysis also shows the proper sequence for both chains. The protein migrates at a molecular weight of ∼30,000 daltons. It has a specific activity of ∼170,000 PU/mg 9225,000 IU/mg), assuming 1 mg/ml has an OD₂₈₀ of 1.3.

### Q. Assays for Detection of Expression of Urokinase

### 1. Chromogenic Substrate

### a. Theory

The assay is based on the proteolytic cleavage of a tripeptide from a chromophoric group. The rate of cleavage can be directly related to the specificity and to the concentration of the protease being tested. Urokinase cleaves the chromogenic substrate S2444 (purchased from Kabi Group Inc., Greenwich, CT). By monitoring the generation of the chromophore, one can determine the amount of functional urokinase present in a sample. Urokinase is synthesized as an inactive precurser form, with activation occurring via the cleavage between residue 26 (lysine) and 27 (isoleucine) (numbering based on the protease clone, Figure 2A). Some preparations of urokinase were found to have been autoactivated and/or were activated by E. coli proteases. To insure activation of urokinase, allowing detection by this chromogenic technique, treatment of the sample with low amounts of trypsin is required. Trypsin is a protease which can cleave the lysine-isoleucine bond required for urokinase activation. However, trypsin can also cleave the chromogenic substrate, and therefore must be eliminated from the assay. Soybean trypsin inhibitor (STI) is a protein which will inactivate trypsin while having no effect on urokinase. Therefore, the assay consists of trypsin activation of urokinase, STI inhibition of the trypsin, and, finally, addition of the chromogenic substrate to measure the functional urokinase present.

### b. Procedure

The assay is performed as follows: 0.2mL of 0.1M Tris, pH 8.0, 50µL of the sample to be assayed, and 5µL of trypsin (0.1mg/mL in 0.1M Tris, pH 8.0 plus 0.25M CaCl₂) were added to a test tube and the sample incubated for 10 minutes at 37°C. The trypsin was inactivated by the addition of 2µL of 10mg/mL STI (in 0.1M Tris, pH 8.0). Urokinase activity was determined by adding 50µL of a 1mM solution of S2444 (in water) and incubating the reaction for 10 minutes at 37°C. Acetic acid (50µL) was added to stop the reaction, the solution centrifuged to remove a precipitate, and the absorbance at 405nm was determined. The actual amount of urokinase can be calculated by comparison of a sample with the reading obtained by performing the assay with dilutions of a standard solution of a known amount of urokinase (obtained from Calbiochem, San Diego, CA).

### 2. Direct Assay of Plasmin Formation

### a. Theory

A much more sensitive assay for urokinase can be obtained by monitoring the urokinase catalyzed conversion of plasminogen to plasmin. Plasmin is an enzyme for which there are chromogenic substrate assays based on the same principles as described in 1 above. The basis of the assay is the determination of the amount of plasmin formed following incubation of the urokinase containing solution with a solution of plasminogen. The greater the amount of urokinase, the greater the amount of plasmin formed.

### b. Procedure

An aliquot of the sample is mixed with 0.10 ml of 0.7 mgs/ml plasminogen (in 0.5M Tris^{·}HCl, pH 7.4, containing .012 M NaCl) and the volume adjusted to 0.15 ml. The mixture is incubated at 37°C for various times (as indicated), 0.35 ml of S2251 (1.0 mM solution in above buffer) is added and the reaction continued for 5 minutes at 37°C. Acetic acid (25 µL) is added to terminate the reaction and absorbance at 405 nm is measured. Quantitation of the amount of activity is obtained by comparison with dilutions of a standard urokinase solution.

### 3. Indirect Assay of Plasmin Formation

### a. Theory

A sensitive assay for urokinase activity has been developed (61). The assay is based on determination of plasmin formation by measuring the extent of plasmin digestion of fibrin in an agar plate containing fibrin and plasminogen. Plasmin produces a clear lysis zone in the fibrin plate. The area of this lysis zone can be correlated to the amount of urokinase in the sample.

### b. Procedure

Following the procedure of Granelli-Piperno and Reich (61), the plates were incubated one to three hours at 37°C and lysis zones measured. Quantitation was obtained by performing the assay with dilutions of a standard urokinase solution.

### R. Detection of Urokinase Activity

### 1. Bacterial Growth and Urokinase Sample Preparaton

A strain of E. coli (W3110) was transformed using a plasmid (pUK33trpLEs) containing a urokinase fusion protein. This expression vehicle (short trpLE fusion) was described above. The cells were grown on minimal media overnight, to an O.D. at 550nm of 1.2. An additional 200mL of media was added. Indole acrylic acid, a compound which appears to enhance expression of the tryptophan operon controlled genes, was added to a concentration of 10 µg/mL. The cells were incubated 2 hours and harvested. The cells obtained from 400mL of media were suspended in water and guanidine was added to a concentration of 7M (final volume of 40 mL). The solution was incubated for 90 minutes at room temperature. Insoluble material was removed by centrifugation. The supernatant was dialyzed against 0.01 M Tris^{·}HCl, pH 7.5, containing 0.1 M NaCl for 4 hours. Insoluble material was removed by centrifugation and the sample was dialyzed for 2.5 hours against 0.01 M Tris^{·}HCl, pH 7.5.

The sample was applied to a 3.9 x 9 cm DE-52 column, which had been equilibrated with 0.01 M Tris^{·}HCl, pH 7.5, and the column washed with the same buffer and eluted with 0.01 M Tris^{·}HCl, pH 7.5, containing 0.15 M NaCl. The peak of activity was pooled and used for all further studies.

### 2. Activity Detection

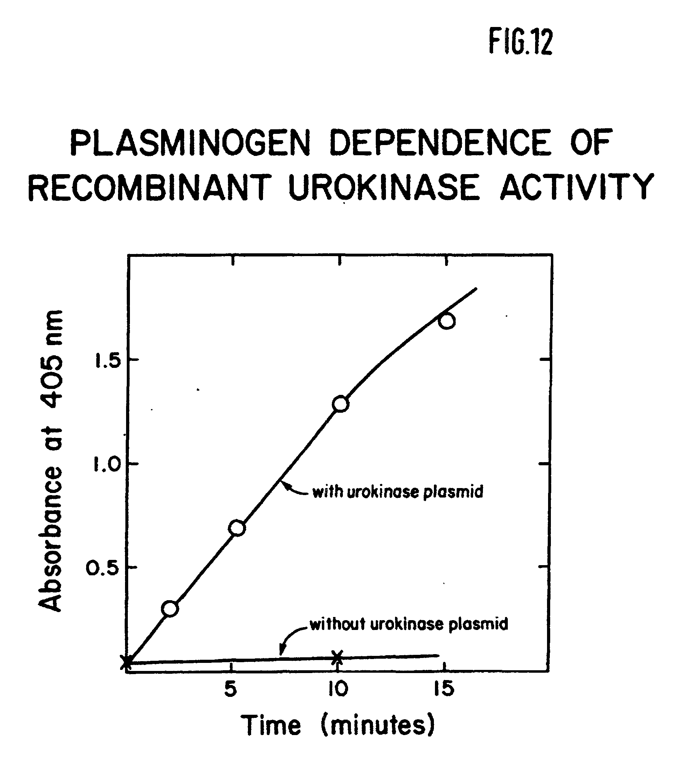

Figure 12 shows the results of the direct activation of plasminogen by fractionated E. Coli extracts when assayed under conditions similar to that described in Section Q.2. supra. An activity is generated which is dependent on the presence of plasminogen. Therefore, the activity being monitored is a plasminogen dependent activity. The activity being measured also increases with time, indicating a time dependent, catalytic generation of plasmin. These properties are consistent with those of urokinase, i.e., a catalytic activation of plasminogen. Similar extraction conditions performed on E. coli which do not contain the urokinase plasmid do not activate plasminogen under these conditions.

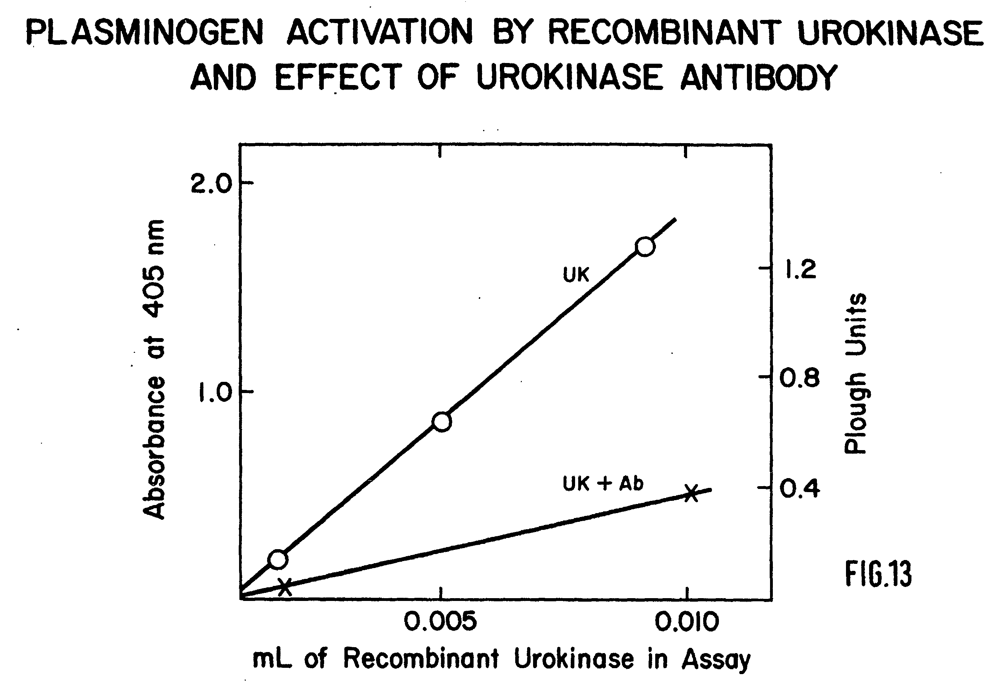

The extracts also were tested in the assays as described above in order to detect and quantitate urokinase activity. Figure 13 shows the effect of various amounts of the bacterially derived fractions in the assay described in Section Q.2 with a 10 minute activation. The values obtained are compared to those obtained using a standard urokinase solution (Plough Unitage determination). The extent of plasminogen activation is directly proportional to the amount of added cloned urokinase. Antibodies raised against purified natural urokinase are known to decrease the activity of natural urokinase. The effect of these antibodies when added to this assay at time 0 are also shown in Figure 13. A marked inhibition of the E. coli derived material is observed. This proves that the activity observed in the E. coli derived material has the same antigenic sites as natural urokinase and therefore that it is indeed urokinase being microbially synthesized.

Similar results for activity detection and antibody inhibition are observed using the fibrin plate assay (described in Section Q.3). These results are summarized in Table I.

**TABLE I**

| FIBRIN PLATE ASSAY OF UROKINASE PROTEIN | | | |
|---|---|---|---|
| SAMPLE | Activity (Plough Units/mL)¹ | Activity in presence of urokinase antibody (Plough Units/mL)¹ | Percent Inhibition |
| Urokinase standard | 112 | 2.5 | 98 |
| | 11 | 0.45 | 96 |
| | 1.1 | 0 | 100 |
| Urokinase produced herein | 480 | 1.12 | 99 |
| | 224 | 0 | 100 |

| | | | |
|---|---|---|---|
| ¹ Standard curve obtained by addition of a known amount of urokinase standard to wells. Values obtained for E. coli fractions and antibody inhibition obtained by extrapolation from this standard curve. | | | |

The standard urokinase activity was inhibited 96 percent or greater by the addition of urokinase antibodies raised against this protein. Assay of the E. coli derived extracts had significant urokinase activity. This activity was almost completely inhibited when antibodies against natural urokinase were added to the assay.

The third assay (the chromogenic substrate assay) also detected a urokinase-like activity in the E. coli extracts. Since it is the least sensitive of the assays, antibody inhibition studies could not be performed due to the large quantities of antibody required to see an inhibition.

The above three assays were quantitated using the standard urokinase purchased from Calbiochem. The values obtained (Plough units per mL) were all of the same order of magnitude: 500 for fibrin plate; 100 for plasminogen activation; and 350 for the chromogenic substrate (S2444). Variations occuring are doubtless due to the relatively impure nature of the material at the time of testing.

### Pharmaceutical Compositions

The human urokinase product can be formulated according to known methods to prepare pharmaceutically useful compositions, whereby the human urokinase product hereof is combined in admixture with a pharmaceutically acceptable carrier vehicle. Suitable vehicles and their formulation are described for example in Remington's Pharmaceutical Sciences by E.W. Martin, which is hereby incorporated by reference. Such compositions will contain an effective amount of the protein hereof together with a suitable amount of vehicle in order to prepare pharmaceutically acceptable compositions suitable for effective administration to the host.

### A. Parenteral Administration

The human urokinase hereof may be parenterally administered to subjects suffering from thromboembolic diseases or conditions. Dosage and dose rate may parallel those currently in use in clinical applications of other cardiovascular, thrombolytic agents, e.g., about 4400 IU/kg body weight as an intravenous priming dose followed by a continuous intravenous infusion at about 4400 IU/kg/hr, for 12 hours, in patients suffering from pulmonary embolism.

As one example of an appropriate dosage form for essentially homogeneous human urokinase in parenteral form applicable herein, a vial containing 250000 IU urokinase activity, 25 mg. mannitol and 45 mg. sodium chloride, may be reconstituted with 5 ml sterile water for injection and admixed with a suitable volume of 0.9 percent Sodium Chloride Injection or 5 percent Dextrose Injection for intravenous administration.

The human urokinase protein hereof has been defined by means of determined DNA gene and deductive amino acid sequencing. It will be understood that natural allelic variations exist and occur from individual to individual. These variations may be demonstrated by (an) amino acid difference(s) in the overall sequence or by deletions, substitutions, insertions, inversions or additions of (an) amino acid(s) in said sequence. In addition, the potential exists in the use of recombinant DNA technology for the preparation of various human urokinase derivatives, variously modified by resultant single or multiple amino acid substitutions, deletions, additions or replacements, for example, by means of site directed mutagenesis of the underlying DNA.

Derivatives of human urokinase having such modifications and allelic variations may be produced in accordance with the invention as defined in the claims, so long as the essential, characteristic human urokinase activity remains unaffected in kind.

Notwithstanding that reference has been made to particular preferred embodiments, it will be further understood that the present invention is not to be construed as limited to such, rather to the lawful scope of the appended claims.

### Bibliography

1. United States Patent No. 3355361.
2. United States Patent No. 3926727.
3. United States Patent No. 4029767.
4. United States Patent No. 4258030.
5. United States Patent No. 4271150.
6. European Patent Application Publn. No. 0037687.
7. United States Patent No. 3555000.
8. Wallen, P., Proc. Serono Symp. 9, 91 (1977).
9. Thorsen, S., et al., Thrombos. Diathes. haemorrh. 28, 65 (1972).
9A. Barnett and Baron, Proc. Soc. Exptl. Biol. 102, 308 (1959).
9B. Banlow and Lazer, Thrombosis Res. 1, 201 (1972).
10. Husain, S.S., et al., Thrombasis al Hemostasis 46, 11 (1981).
10A. Wun et al., J. Biol. Chem. 257, 3276 (1982).
11. Ratzkin et al., Proc. Natl. Acid. Sci. (USA) 78, 3313 (1981).
11A. Bollen, A. et al., Biochem. Biophys. Res. Commun. 103, 391 (1981).
12. British Patent Application Publn. No. 2007676A.
13. Wetzel, American Scientist 68, 664 (1980).
14. Microbiology, 2d Ed., Harper and Row Publications, Inc., Hagerstown, Maryland (1973), esp. pp. 1122 et seq.
15. Scientific American 245, 66 et seq. (1981).
16. British Patent Application Publn. No. 2055382A.
17. German Offenlegungsschrift 2644432.
18. Chang et al., Nature 275, 617 (1978).
19. Itakura et al., Science 198, 1056 (1977).
20. Goeddel et al., Nucleic Acids Research 8, 4057 (1980).
21. European Patent Application Publn. No. 0035776.
22. Siebenlist et al., Cell 20, 269 (1980).
23. Stinchcomb et al., Nature 282, 39 (1979).
24. Kingsman et al., Gene 7, 141 (1979).
25. Tschumper et al., Gene 10, 157 (1980).
26. Mortimer et al., Microbiological Reviews 44, 519 (198 ).
27. Miozzari et al., Journal of Bacteriology 134, 48 (1978).
28. Jones, Genetics 85, 23 (1977).
29. Hitzeman, et al., J. Biol. Chem. 255, 12073 (1980).
30. Holland et al., Biochemistry 17, 4900 (1978).
31. Tissue Culture, Academic Press, Kruse and Patterson eds, (1973).
32. Gluzman, Cell 23, 175 (1981).
33. Lusky et al., Nature 293, 79 (1981).
34. Gluzman et al., Cold Spring Harbor Symp. Quant. Biol. 44, 293 (1980).
35. Fiers et al., Nature 273, 113 (1978).
36. Reddy et al., Science 200, 494 (1978).
37. Bolivar et al., Gene 2, 95 (1977).
38. Vetterlein et al., J. Biol. Chem. 255, 3665 (1980).
39. Eagle, H., Science 130, 432 (1959).
40. Lynch et al, Virology 98, 251 (1979).
41. Aviv et al., Proc. Natl. Acad. Sci. USA 69, 1408 (1972).
42. Lehrach et al., Biochemistry 16, 4743 (1977).
43. Jackson et al., Proc. Natl. Acad. Sci. (USA) 74, 5598 (1977).
44. Goeddel et al., Nature 281, 544 (1979).
45. Wickens et al., J. Biol. Chem. 253, 2483 (1978).
46. Chang et al., Nature 275, 617 (1978).
47. Crea et al., Proc. Natl. Acad. Sci. (USA) 75, 5765 (1978).
47A. European Patent Application Publication No. 0036776.
48. Miller, Experiments in Molecular Genetics, p. 431-3, Cold Spring Harbor Lab., Cold Spring Harbor, New York (1972).
49. Grunstein et al., Proc. Natl. Acad. Sci. U.S.A. 72, 3961 (1975).
50. Wallace et al., Nucleic Acids Research 9, 879 (1981).
51. Birnboim et al., Nucleic Acids Research 7, 1513 (1979).
52. Smith, Methods Enzymol. 65, 560 (1980).
53. Messing et al., Nucleic Acids Res. 9, 309 (1981).
54. Taylor et al., Biochem. Biophys. Acta 442, 324 (1976).
55. Fritsch et al., Cell 19, 959 (1980).
56. Goeddel et al., Nature 290, 20 (1981).
57. Blobel et al., Biomembranes, Vol. 2, ed. Manson, pp. 193, Plenum.
58. Goeddel et al., Nature 287, 411 (1980).
59. Itakura et al., Science 198, 1056 (1977).
60. Bingham et al., Nucleic Acids Research 5, 3457 (1978).
61. Granelli-Piperino and Reich, J. Exp. Med. 148, 223.
62. Crowley et al., Mol. and Cellular Biol. 3, 44 (1983).

## Claims

1. A process for producing an expression vehicle which comprises operably linking a first DNA sequence to a second DNA sequence capable of effecting expression of said first DNA sequence in a selected host cell capable of producing glycoproteins wherein said first DNA sequence encodes a polypeptide having an intact lysine - isoleucine bond at the position corresponding to amino acids 158 - 159 in the native prourokinase molecule, having sufficient of the amino acid sequence set out in Figures 4A and 4B such that, on activation, the activated glycosylated polypeptide exhibits the enzymatic activity of human urokinase, and optionally having an amino acid or an amino acid sequence preceding the N-terminus of the ordinarily first amino acid of said polypeptide.

2. A process according to claim 1 wherein a methionine residue precedes the N-terminus.

3. A process according to claim 1 or 2 wherein the polypeptide is one having glycosylation different from that of native urokinase.

4. A process according to any one of the preceding claims wherein the polypeptide expressed is one having the sequence Lys-Ile-Ile-Gly-Gly, at positions corresponding to amino acids 158-162 in the native prourokinase molecule.

5. A process according to any one of the preceding claims wherein the expressed polypeptide is one having the amino acid sequence or substantially the amino acid sequence as depicted in Figs 4A and 4B.

6. A process to any one of claims 1 to 4 wherein the polypeptide is one having the amino acid sequence as depicted in Figs 2A and B and derivatives of said amino acid sequence which still comprises the enzymatic portion of human urokinase.

7. A process according to any one of the preceding claims wherein the resulting expression vehicle is capable, in a transformant microorganism or cell culture, of expressing the first DNA sequence in a selected host cell.

8. A process for producing a microorganism or cell culture capable of expressing a selected polypeptide which comprises transforming a microorganism or vertebrate cell with an expression vehicle produced by a process according to any one of the preceding claims.

9. A process according to claim 8 wherein the microorganism is a yeast or a vertebrate cell and a glycosylated polypeptide is obtained.

10. A plasmid selected from the group consisting of pUK33trpLEL, pUK33trpLEs, pUK33trp103, pUK54trp207-1 and ppreUK54trp207-1, wherein said plasmids are as producible according to the construction schemes set out in Figs. 5, 6, 7, 9 and 10, respectively.

11. A yeast or a vertebrate cell transformed with any one of the plasmids according to claim 10.

12. A process for producing a glycosylated polypeptide comprising the step of culturing a microorganism or cell culture produced by a process according to claim 8 or claim 9 under conditions such that the polypeptide is expressed, and optionally recovering the glycosylated polypeptide from the culture medium.

## Patentansprüche

1. Verfahren zur Herstellung eines Expressionsvehikels, welches das operable Verbinden einer ersten DNA-Sequenz mit einer zweiten DNA-Sequenz umfasst, die fähig ist, die Expression der ersten DNA-Sequenz in einer ausgewählten Wirtszelle herbeizuführen, die fähig ist, Glykoproteine zu produzieren, worin die erste DNA-Sequenz für ein Polypeptid mit einer intakten Lysin-lsoleucin-Bindung an jener Position kodiert, die den Aminosäuren 158 bis 159 im nativen Prourokinasemolekül entspricht, das eine ausreichende Menge der in den Fig. 4A und 4B dargelegten Aminosäuresequenz aufweist, so dass nach Aktivierung das aktivierte glykosylierte Polypeptid die enzymatische Aktivität von Human-Urokinase aufweist, und das gegebenenfalls eine Aminosäure oder eine Aminosäuresequenz aufweist, die dem N-Terminus der üblicherweise ersten Aminosäure des Polypeptids vorangeht.

2. Verfahren nach Anspruch 1, worin dem N-Terminus ein Methioninrest vorangeht.

3. Verfahren nach Anspruch 1 oder 2, worin das Polypeptid eines ist, dessen Glykosylierung sich von jener von nativer Urokinase unterscheidet.

4. Verfahren nach einem der vorangegangenen Ansprüche, worin das exprimierte Polypeptid eines mit der Sequenz Lys-Ile-Ile-Gly-Gly an Positionen ist, die den Aminosäuren 158 bis 162 im nativen Prourokinase-Molekül entsprechen.

5. Verfahren nach einem der vorangegangenen Ansprüche, worin das exprimierte Polypeptid eines mit der Aminosäuresequenz oder im Wesentlichen der Aminosäuresequenz wie in den Fig. 4A und 4B dargestellt ist.

6. Verfahren nach einem der Ansprüche 1 bis 4, worin das Polypeptid eines mit der Aminosäuresequenz wie in den Fig. 2A und B dargestellt und von Derivaten der Aminosäuresequenz, die weiterhin den enzymatischen Abschnitt von Human-Urokinase umfassen, ist.

7. Verfahren nach einem der vorangegangenen Ansprüche, worin das resultierende Expressionsvehikel fähig ist, in einem bzw. einer transformierten Mikroorganismus oder Zellkultur die erste DNA-Sequenz in einer ausgewählten Wirtszelle zu exprimieren.

8. Verfahren zur Herstellung eines Mikroorganismus oder einer Zellkultur, der bzw. die zum Exprimieren eines ausgewählten Polypeptids fähig ist, welches das Transformieren eines Mikroorganismus oder einer Wirbeltierzelle mit einem Expressionvehikel umfasst, das nach einem Verfahren nach einem der vorangegangenen Ansprüche erzeugt wurde.

9. Verfahren nach Anspruch 8, worin der Mikroorganismus eine Hefe oder eine Wirbeltierzelle ist und ein glykosyliertes Polypeptid erhalten wird.

10. Plasmid, ausgewählt aus der aus pUK33trpLEL, pUK33trpLEs, pUK33trp103, pUK54trp207-1 und ppreUK54trp207-1 bestehenden Gruppe, worin die Plasmide nach den in den Fig. 5, 6, 7, 9 bzw. 10 dargelegten Konstruktionsschemata erzeugt werden können.

11. Hefe oder Wirbeltierzelle, die mit einem der Plasmide nach Anspruch 10 transformiert ist.

12. Verfahren zur Herstellung eines glykosylierten Polypeptids, das den Schritt des Kultivierens eines Mikroorganismus oder einer Zellkultur, der bzw. die nach einem Verfahren nach Anspruch 8 oder 9 hergestellt wurde, unter solchen Bedingungen, dass das Polypeptid exprimiert wird, und gegebenenfalls des Gewinnens des glykosylierten Polypeptids aus dem Kulturmedium umfasst.

## Revendications

1. Procédé pour la production d'un vecteur d'expression qui comprend la liaison fonctionnelle d'une première séquence d'ADN à une seconde séquence d'ADN apte à effectuer l'expression de ladite première séquence d'ADN dans une cellule hôte choisie capable de produire des glycoprotéines, dans lequel ladite première séquence d'ADN code pour un polypeptide ayant une liaison lyse-isoleucine intacte à la position correspondant aux amino-acides 158-159 dans la molécule de pro-urokinase naturelle, ayant une quantité suffisante de la séquence d'amino-acides représentée sur les figures 4A et 4B de telle sorte que, par activation, le polypeptide glycosylé activé présente l'activité enzymatique de l'urokinase humaine, et ayant facultativement un amino-acide ou une séquence d'amino-acides précédant l'extrémité N-terminale du premier amino-acide habituel dudit polypeptide.

2. Procédé suivant la revendication 1, dans lequel un résidu méthionine précède l'extrémité N-terminale.

3. Procédé suivant la revendication 1 ou 2, dans lequel le polypeptide est un polypeptide ayant une glycosylation différente de celle de l'urokinase naturelle.

4. Procédé suivant l'une quelconque des revendications précédentes, dans lequel le polypeptide exprimé est un polypeptide ayant la séquence Lys-Ile-Ile-Gly-Gly, aux positions correspondant aux amino-acides 158-162 dans la molécule de pro-urokinase naturelle.

5. Procédé suivant l'une quelconque des revendications précédentes, dans lequel le polypeptide exprimé est un polypeptide ayant la séquence d'amino-acides ou subtantiellement la séquence d'amino-acides représentée sur les figures 4A et 4B.

6. Procédé suivant l'une quelconque des revendications 1 à 4, dans lequel le polypeptide est un polypeptide ayant la séquence d'amino-acides représentée sur les figures 2A et 2B et des dérivés de ladite séquence d'amino-acides comprenant encore la partie enzymatique de l'urokinase humaine.

7. Procédé suivant l'une quelconque des revendications précédentes, dans lequel le vecteur d'expression résultant est capable, dans une culture de micro-organismes transformants ou de cellules transformantes, d'exprimer la première séquence d'ADN dans une cellule hôte choisie.

8. Procédé pour la production d'une culture de micro-organismes ou de cellules capable d'exprimer un polypeptide choisi, qui comprend la transformation d'un micro-organisme ou d'une cellule de vertébré avec un vecteur d'expression produit par un procédé suivant l'une quelconque des revendications précédentes.

9. Procédé suivant la revendication 8, dans lequel le micro-organisme est une levure ou une cellule de vertébré et un polypeptide glycosylé est obtenu.

10. Plasmide choisi dans le groupe consistant en pUK33trpLEL, pUK33trpLEs, pUK33trp103, pUK54trp207-1 et ppreUK54trp207-1, lesdits plasmide pouvant être produits suivant les schémas de construction représentés, respectivement, sur les figures 5, 6, 7, 9 et 10.

11. Levure ou cellule de vertébré, transformée avec l'un quelconque des plasmides suivant la revendication 10.

12. Procédé pour la production d'un polypeptide glycosylé, comprenant l'étape consistant à cultiver une culture de micro-organismes ou de cellules produite par un procédé suivant la revendication 8 ou la revendication 9 dans des conditions telles que le polypeptide soit exprimé et, facultativement, l'étape consistant à recueillir le polypeptide glycosylé à partir du milieu de culture.
